(19) Europäisches Patentamt / European Patent Office / Office européen des brevets

(11) EP 2 998 334 B1

(12) EUROPÄISCHE PATENTSCHRIFT

(45) Veröffentlichungstag und Bekanntmachung des Hinweises auf die Patenterteilung:
**25.03.2020 Patentblatt 2020/13**

(21) Anmeldenummer: **15401094.6**

(22) Anmeldetag: **08.09.2015**

(51) Int Cl.:
*C08G 18/76* (2006.01) *C04B 24/28* (2006.01)
*C04B 26/16* (2006.01) *C07D 317/36* (2006.01)
*C08G 18/80* (2006.01) *C09K 8/42* (2006.01)
*C08G 18/28* (2006.01) *C08G 71/04* (2006.01)

(54) **HÄRTBARES KUNSTHARZ MIT ERHEBLICHEN ANTEILEN AN CYCLISCHEN CARBONATGRUPPEN, SOWIE/UND CYCLOCARBONATHARZ-BASIERTE BEFESTIGUNGSSYSTEME, DEREN HERSTELLUNG UND VERWENDUNG**

CURABLE RESIN WITH SIGNIFICANT LEVELS OF CYCLIC CARBONATE GROUPS, AND CYCLOCARBONATE RESIN BASED FIXING SYSTEMS, THEIR PREPARATION AND USE

RESINE SYNTHETIQUE DURCISSABLE A FORTE PROPORTION DE GROUPES CARBONATE CYCLIQUES ET SYSTEMES DE FIXATION A BASE DE RESINE DE CYCLOCARBONATE, SA FABRICATION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2014 DE 102014013989**
**13.08.2015 DE 102015113351**

(43) Veröffentlichungstag der Anmeldung:
**23.03.2016 Patentblatt 2016/12**

(73) Patentinhaber: **fischerwerke GmbH & Co. KG**
**72178 Waldachtal (DE)**

(72) Erfinder:
- **Grün, Jürgen**
  **79268 Bötzingen (DE)**
- **Vogel, Martin**
  **79286 Glottertal (DE)**
- **Schlenk, Christian**
  **79211 Denzlingen (DE)**
- **Weinelt, Christian**
  **79312 Emmendingen (DE)**
- **Angarano, Marco**
  **72070 Tübingen (DE)**

(56) Entgegenhaltungen:
**WO-A1-2006/010408** **WO-A1-2014/033045**
**US-A1- 2010 137 507**

EP 2 998 334 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft die Verwendung eines Kunstharz-Befestigungssystem auf Basis cyclischer Carbonatharze zum Befestigen von Verankerungselementen in Bohrlöchern oder Spalten, sowie Härtbare Kunstharze mit di- und trifunktionellen Reaktiwerdünnern, wie jeweils in den unabhängigen, vorzugsweise in Kombination mit mindestens einem abhängigen Anspruch, definiert.

Es ist bekannt, Kunstharzmassen auf Basis ungesättigter Polyesterharze oder Vinylesterharze oder auf Epoxidbasis als Klebemittel für die Verankerung in der chemischen Befestigungstechnik, beispielsweise zur Fixierung von Verankerungsmitteln in Bohrlöchern, zu verwenden. Häufig finden hierbei Zwei-Komponentensysteme Einsatz, deren eine Komponente ein Reaktivharz und deren andere Komponente einen Härter beinhaltet. Durch Vermischen der beiden Komponenten werden die Reaktionen, die zur Aushärtung führen, in Gang gebracht.

Durch Polyaddition härtende Befestigungssysteme auf Polyurethanbasis sind für die Befestigung von beispielsweise Verankerungselementen in Bohrlöchern als sogenannte "chemische Dübel" grundsätzlich ebenfalls bekannt, siehe beispielsweise die Injektionsmasse fill & fix®, ein lösemittelfreies Zwei-Komponentensystem auf Polyurethanbasis der fischerwerke GmbH & Co. KG, welches ein solches - zur Bildung von Polyurethanen übliches - isocyanatbasiertes Reaktivharz in der einen Komponente und den entsprechenden Härter in der anderen Komponente enthält.

Ein Nachteil zumindest einiger Systeme auf Basis von Zwei-Komponenten-Polyurethan-Klebstoffen ist die Toxikologie von monomeren Isocyanaten, insbesondere leicht flüchtigen und/oder leicht migrierenden monomeren Diisocyanaten. Die Anwendung von Produkten mit einem hohen Gehalt an leicht flüchtigen Diisocyanaten erfordert seitens des Anwenders aufwendige Arbeitsschutzmaßnahmen, insbesondere durch die höchstzulässige Konzentration von Arbeitsstoffen als Gas, Dampf oder Schwebstoff in der Luft.

Diese fortschreitenden Einschränkungen - sowohl für den Hersteller als auch für den Endnutzer - bei der Verwendung von Isocyanaten (Toxizität der Isocyanate und auch damit einhergehendes Selbstbedienungsverbot) als Reaktionspartner für z.B. Polyole bei der Herstellung von Kunstharz- bzw. Befestigungssystemen, beispielsweise im Rahmen der REACH-Verordnung in der Europäischen Union, machen die Suche nach Alternativen auch aus ökologischen Gründen wünschenswert und erforderlich.

Eine Alternative zu isocyanatbasierten Polyurethanen bilden die isocyanatfreien Polyurethane, oft mit NIPU - non isocyanate polyurethane - abgekürzt. Isocyanatfreie Polyurethane werden - wie der Name bereits impliziert - ohne die Verwendung toxischer Isocyanate hergestellt. Bekannt ist beispielsweise die Herstellung von isocyanatfreien Polyurethanen aus cyclische Carbonatgruppen aufweisenden Dicarbonaten und Diaminen, die zu Polymeren führen, die in der $\beta$-Position zur Urethangruppe herstellungsbedingt Hydroxygruppen enthalten.

Cyclische Carbonate werden beispielsweise durch Umesterung von Kohlensäureestern, wie z.B. Dimethylcarbonat und/oder Ethylencarbonat, mit Polyolen erhalten, wobei die Polyole bevorzugt mindestens drei und insbesondere bevorzugt vier oder mehr Hydroxylgruppen tragen, von denen je zwei mit Kohlensäureestern in einer Umesterung zu cyclischen Fünf- oder Sechsring-Carbonaten reagieren. Als mehrwertige Polyole sind beispielsweise zu nennen: Diglycerin, Triglycerin, Polyglycerin, Zuckeralkohole (z.B. Xylit, Mannit, Erytherit), Di- und Trimethylolpropan, Di- und Trimethylolethan, Pentaerythrit, Dipentaerythrit und Glycerin. Die Herstellung der cyclischen Carbonate aus den Polyolen geschieht in dem Fachmann geläufiger Weise, insbesondere durch Umsetzung der Polyole mit den Carbonaten im stöchiometrischen Verhältnis 1,0 : 1,0 bis 1,0 : 10,0 (Verhältnis von 1,2- oder 1,3-Glykolgruppen zu Carbonatgruppen), insbesondere unter Katalyse.

Bevorzugt werden die cyclischen Carbonate durch Umsetzung von Kohlendioxid ($CO_2$) mit Epoxidverbindungen nach bekannter Art und Weise erhalten. Dies führt nicht nur zu einer direkten Nutzung von $CO_2$ als $C_1$-Kohlenstoffquelle in der Herstellung von industriellen Produkten und Massenchemikalien, sondern wirkt auch der globalen Klimaerwärmung entgegen, wofür bekanntlich der fortwährende Anstieg des $CO_2$-Gehaltes in der Erdatmosphäre und der damit verbundene Treibhauseffekt anerkannte Motoren sind. Derartige Umsetzungen sind beispielsweise in der DE 35 29 263, DE 36 00 602 und in WO 84/03701 beschrieben.

Erste Versuche zur Verwendung von solchen Cyclocarbonatharzen (cyclische Carbonatgruppen aufweisende Harze) sind bekannt. So beschreibt beispielsweise die US 3,072,613 als Klebstoff einsetzbare harzartige Polyurethanprodukte, die durch Reaktion eines multifunktionellen cyclischen Carbonates mit einem polyfunktionellen Amin erhalten werden und eine reduzierte Viskosität von größer gleich 0,12 als 0,2 Gew.-%ige Lösung in Dimethylformamid aufweisen. Die multifunktionellen cyclischen Carbonate werden durch Umsetzung von monomeren Verbindungen, beispielsweise durch Umsetzung von 4,4'-Diphenylmethandiisocyanat mit Glycerincarbonat, erhalten.

Die Synthese cyclische Carbonatgruppen enthaltender Oligourethane aus Diisocyanaten oder Polyisocyanaten unter Verwendung von TMP-Carbonat und ihre Verwendung als Vernetzer für Polyole in thermisch härtbaren Beschichtungssystemen ist auch aus EP-A 0 703 230 bekannt.

Nachteilig an den cyclischen Carbonatgruppen aufweisenden Umsetzungsprodukten gemäß den genannten Schriften ist, dass die Produkte als Feststoff anfallen oder zumindest extrem hohe Viskositäten mit teilweiser Kristallisationsneigung aufweisen. Ein weiterer Nachteil ist die Notwendigkeit der thermischen Härtung. Diese Nachteile machen

die cyclischen Carbonatgruppen aufweisenden Produkte für den Einsatz zur Verwendung als lösemittelfreies, kalthärtendes Mehrkomponentensystem wie z.B. Kartuschen-Injektionssystem - für die Befestigung von Verankerungsmitteln in Bohrlöchern oder Spalten - ungeeignet.

Die WO 2006/010408 beschreibt isocyanatgruppenfreie Umsetzungsprodukte linearer Polyurethanpräpolymere auf Basis von Diphenylmethandiisocyanat (MDI) mit Glycerincarbonat, welche ein mittleres Molekulargewicht ($M_n$) von ≥ 1000 g/mol aufweisen. Die Umsetzungsprodukte lassen sich mit Verbindungen, die mindestens zwei primäre oder sekundäre Aminogruppen tragen, bereits bei Raumtemperatur vernetzen. Solche Zweikomponenten-Bindemittel finden als Kleb- und Dichtstoffe, insbesondere als Kaschierklebstoff für Verbundfolien, Verwendung. Ein Einsatz dieser Umsetzungsprodukte zur Verwendung als kalthärtendes Kartuschen-Injektionssystem in der Befestigungstechnik ist allerdings schon aufgrund der hohen Viskositäten nicht möglich (siehe Beispiel 9).

Die US 2010/0137507 beschreibt modifizierte (Poly)isocyanat-Zusammensetzungen zur Verwendung als Anstreichmittel und Lacke.

Die WO 2014/033045 beschreibt die Herstellung von Polyurethanen, die cyclische Carbonatstrukturen beinhalten, sowie die Verwendung dieser Polymeren als Bindemittel in Dispersionen als Lack-, Dichtstoff- oder Klebrohstoffe.

Vor diesem Hintergrund besteht die Aufgabe, alternative durch Polyaddition härtende Kunstharzsysteme auf Polyurethanbasis für den Bereich der Befestigungstechnik, insbesondere der erfindungsgemäßen Verwendung zur Befestigung von Verankerungsmitteln in Löchern oder Spalten, zur Verfügung zu stellen, die einerseits nicht auf die Verwendung von toxischen Isocyanaten mit den vorgenannten Nachteilen angewiesen sind und andererseits weitere vorteilhafte Eigenschaften aufweisen, wie nachfolgend dargelegt, wie zum Beispiel den Einsatz eines - zur Bildung von NIPU bekannten - cyclischen Carbonatharzes zur Verwendung als kalthärtendes Kartuschen-Injektionssystem in der chemischen Befestigungstechnik, beispielsweise zur Fixierung von Verankerungsmitteln in Bohrlöchern, zu ermöglichen.

Es wurde nun gefunden, dass es möglich ist, Kunstharz-Befestigungssysteme bereitzustellen, die auf Rohstoffen basieren, welche gleichermaßen durch chemische Fixierung von $CO_2$ an mehrfunktionellen Epoxidverbindungen und/oder durch Umesterung von Kohlensäureestern mit Polyolen cyclische Carbonatgruppen enthalten. Vorzugsweise werden die Rohstoffe bzw. cyclischen Carbonatharze (=Cyclocarbonatharze) durch Umsetzung von Isocyanaten mit hydroxyfunktionellen cyclischen Carbonaten, welche mindestens eine cyclische Carbonatgruppe enthalten, hergestellt. Hierdurch können mit - aus dem Bereich auch der Epoxidhärtung bekannten (beispielsweise) - Aminen, isocyanatfreie bzw. alternative durch Polyaddition härtende Befestigungssysteme auf Polyurethanbasis hergestellt werden.

Als vorteilhafte Eigenschaften sind hier insbesondere die "Isocyanatfreiheit" (Endnutzer kommt nicht mit toxischen Isocyanaten in Berührung) und die allgemein bessere Kennzeichnung der cyclischen Carbonatharze z.B. im Vergleich mit Epoxiden (keine sensibilisierende Wirkung; Einsatz von Glycerincarbonat in Kosmetika), aber auch die Nutzung bzw. chemische Fixierung von $CO_2$ als Rohstoff, wodurch ökologisch sinnvolle neue chemische Befestigungssysteme generiert werden, hervorzuheben.

Es wurde insbesondere überraschend gefunden, dass die genannten vorteilhaften Eigenschaften und der Einsatz eines cyclischen Carbonatharzes mit geeigneten Viskositäten ohne Kristallisationsneigung zur Verwendung als kalthärtendes Kartuschen-Injektionssystem auch mit sehr hohen und verbesserten Verbundspannungen zu vereinbaren sind. Dies wird unter Verwendung von Isocyanaten mit einer Funktionalität von mindestens 1,5 oder größer als 1,5, beispielsweise durch Abmischungen von monomeren (Poly-)Isocyanaten, wie 2,2’- und 4,4’-Diphenylmethandiisocyanat und/oder z.B. p-Toluolsulfonylisocyanat, und höherfunktionellen Polyisocyanaten oder durch Verwendung von polymerem MDI (Roh-MDI), welche höhere Homologe enthalten, als Ausgangsmaterial für die Herstellung von auf cyclischen Carbonatharzen beruhenden und durch Polyaddition härtenden Kunstharz-Befestigungssysstemen in Kombination mit einem oder mehrerer Reaktivverdünner möglich.

Vorteilhaft und erfindungsgemäß kann es zum Beispiel auch sein, solche Isocyanate für die Reaktion mit den hydroxyfunktionellen cyclischen Carbonaten zu verwenden, welche die Polymerdefinition gemäß REACH erfüllen, d.h., dass keine einzelne Molekülspezies zu mehr als 50 Gewichtsprozent vorliegt und gleichzeitig mehr als 50 Gewichtsprozent der Ketten aus mindestens 3n + 1 kovalent gebundenen Monomereinheiten zusammengesetzt sind; und/ oder zumindest solche Isocyanate verwendet werden, welche zusammen mit dem hydroxyfunktionellen cyclischen Carbonat, beispielsweise Glycerincarbonat, als Monomereinheit den Polymerstatus erfüllen. Dies kann sich nicht nur positiv auf die Viskosität auswirken, sondern auch die bereits erwähnte Kristallisationsneigung aufgrund von Wasserstoffbrücken unterdrücken. Beides fördert die Verwendbarkeit des cyclischen Carbonatharzes als lösemittelfreies, kalthärtendes Mehrkomponenten-, z.B. Kartuschen-Injektionssystem im Bereich der Befestigungstechnik.

Bei den erhältlichen bzw. nach Umsetzung erhaltenen cyclischen Carbonaten handelt es sich um solche in Form cyclischer Carbonatharze (was bedeutet, dass die Harze cyclische Carbonatgruppen haben) mit einer durchschnittlichen Funktionalität von 1,5 oder größer als 1,5 , insbesondere von 2,1 bis 5, zum Beispiel von 2,2 bis 4, vorteilhaft z.B. von 2,3 bis 3,5 , cyclischen Carbonatgruppen je Molekül.

Vorteilhaft liegen die Molekulargewichte der cyclischen Carbonate bei ≤ 1000 g/mol (Mittelwert bezogen auf die Molekülzahl). Besonders bevorzugt liegen sie unter 1000 g/mol, insbesondere bei ≤ 990, z.B. ≤ 950, wie bei 800 oder niedriger.

Als Viskositätsbereiche für die cyclische Carbonatkomponente werden vorzugsweise solche im Bereich von 200 bis 400.000 mPas, wie von 400 bis 320.000 mPas bei 23 °C vorgesehen.

Viskositäten werden gemessen mit einem Rheometer AR2000 der Firma TA Instruments mit einer Platte-Platte-Geometrie, Durchmesser 25 mm, Spalt 1000 $\mu$m im Flowversuch mit einem Drehmoment von 50.000 $\mu N \cdot m$ bei 23 oder wie nachfolgend angegeben.

Bei den resultierenden Produkten bei Umsetzung mit einem Härter wie unten dargelegt (beispielsweise in einem Bohrloch) handelt es sich bevorzugt um Ester von Carbamidsäurederivaten (Urethanen) und/oder Thiourethanen.

In einer ersten bevorzugten Ausführungsform betrifft die Erfindung daher die Verwendung eines Kunstharz-Befestigungssystems auf der Basis von ein oder mehreren cyclischen Carbonatharzen mit einer durchschnittlichen Funktionalität von 1,5 oder mehr als 1,5 cyclischen Carbonatgruppen je Molekül zur Befestigung von Verankerungmitteln in Löchern oder Spalten. Vorzugsweise handelt es sich bei den cyclischen Carbonatharzen in allen Ausführungsformen der Erfindung um solche, die nach dem nachfolgend beschriebenen Verfahren herstellbar, insbesondere hergestellt, sind.

Für Referenzzwecke betrifft die vorliegende Offenbarung ein Verfahren zur Herstellung von cyclische Carbonatharze beinhaltenden Kunstharz-Befestigungssystemen, umfassend einen Schritt, bei dem Isocyanate mit einer durchschnittlichen Funktionalität von Isocyanatgruppen von 1,5 oder größer als 1,5 je Molekül mit hydroxylgruppenhaltigen cyclischen Carbonaten (1,3-diox(ol)an-2-one) der Formel I

$$Q\text{-}X\text{-}R^1 \qquad (I)$$

worin:

X unabhängig voneinander einen ein- oder mehrfach verzweigten oder geradkettigen aliphatischen oder heteroaliphatischen Rest mit 1 bis 36 sein Grundgerüst ausbildenden Atomen bedeutet, der gegebenenfalls substituiert, beispielsweise mit Hydroxygruppen, sein kann, wobei heteroaliphatisch bedeutet, dass 1 oder mehr, insbesondere 1 bis 3 Kohlenstoffatome in einem aliphatischen Grundgerüst durch Heteroatome ausgewählt aus O, S und NZ ersetzt sind, worin Z Wasserstoff, $C_1$-$C_7$-Alkyl, Phenyl oder Phenyl-$C_1$-$C_7$-Alkyl bedeutet;

$R^1$ einen Rest der Teilformel A

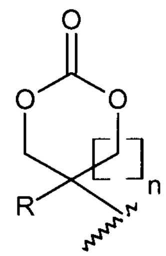

(A)

bedeutet, in dem R für Wasserstoff oder einen gesättigten oder ungesättigten, linearen oder verzweigten, aliphatischen Rest mit 1 bis 7 Kohlenstoffatomen steht, der unsubstituiert oder durch OH substituiert ist;

n für 0 oder 1 steht;

Q für einen Hydroxyrest (OH-) oder einen Rest der Teilformel B

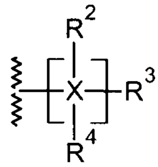

(B)

steht, worin $R^2$, $R^3$ und $R^4$ unabhängig voneinander Wasserstoff, Hydroxy oder einen Substituenten der Teilformel (A) bedeuten, wobei die Variablen R und n der Teilformel (A) gleich oder voneinander unterschiedlich aus den bei der Definition von Teilformel A genannten ausgewählt sind und X die oben genannte Bedeutung hat;

und dass das Molekül der Fomel I unsubstituiert oder substituiert, beispielsweise durch ein bis 5 Reste unabhängig

voneinander ausgewählt aus Cyano, Carboxyl und $C_1$-$C_7$-Alkoxycarbonyl, ist;

vorzugsweise in Gegenwart eines oder mehrerer Reaktivverdünner,

umgesetzt werden und gegebenenfalls die Zugabe weiterer Zusätze sowie insbesondere zusätzlich die Konfektionierung als Mehrkomponentensystem (z. B. Kartuschen-Injektionssystem) mit einem Härter, insbesondere als Mehrkomponentenkit.

Eine weitere Ausführungsform der Erfindung betrifft die Verwendung eines Kunstharz-Befestigungssystem auf der Basis von ein oder mehreren cyclischen Carbonatharzen zur Befestigung von Verankerungsmitteln wie in den Ansprüchen definiert in Bohrlöchern in Gebäudesubstraten, und offenbart ferner für Referenzzwecke Verfahren, die diese Verwendung beinhalten.

Die nachfolgenden Definitionen dienen der Klärung bestimmter Begriffe oder Symbole und der Beschreibung besonderer Ausführungsformen der Erfindung, wobei in den vor- und nachstehend genannten Ausführungsformen der Erfindung einzelne, mehrere oder alle Begriffe oder Symbole durch speziellere Definitionen ersetzt werden können, was zu besonderen Ausführungsformen der Erfindung führt.

Wo Gewichtsangaben in Prozent (stets als Gew.-% zu verstehen) gemacht werden, beziehen sich diese, wenn nichts anderes gesagt ist, auf die Gesamtmasse der Reaktanden und Zusätze des erfindungsgemäßen oder erfindungsgemäß verwendeten Klebemittels (also die in der nach dem Mischen auszuhärtenden Masse vorhandenen Bestandteile bzw. deren Vorstufen ohne Verpackung, außer im Falle von Patronen oder Folien, die ebenfalls als Füllstoffe einen Beitrag zur Gesamtmasse des härtenden bzw. gehärteten Materials liefern können, und ohne andere mögliche Teile wie Statikmischer, Kartuschengehäuse oder dergleichen).

"Beinhalten" oder "umfassen" bedeutet, dass neben den genannten Komponenten oder Merkmalen noch andere vorhanden sein können, steht also für eine nicht abschließende Auf-zählung, im Gegensatz zu "enthalten", das eine abschließende Aufzählung der bei seiner Verwendung aufgezählten Komponenten oder Merkmale bedeutet.

Wo das Attribut "ferner" erwähnt wird, bedeutet dies, dass Merkmale ohne dieses Attribut stärker bevorzugt sein können.

"Und/oder" bedeutet, dass die genannten Merkmale/Substanzen jeweils alleine oder in Kombination von zwei oder mehr der jeweils genannten Merkmale/Substanzen vorliegen können.

"Ein" steht in der Regel (außer wenn wie direkt anschließend hier im Satz als Zahl erkennbar) für den unbestimmten Artikel und bedeutet insbesondere "mindestens ein" (im Sinne von 1, 2 oder mehr).

Wo von "hetero" oder "Hetero" im Zusammenhang mit Atomen die Rede ist, bedeutet dies insbesondere 1 bis drei unabhängig voneinander aus N (insbesondere als NZ, worin Z Wasserstoff, $C_1$-$C_7$-Alkyl, Phenyl oder Phenyl-$C_1$-$C_7$-Alkyl bedeutet), O und S ausgewählte Heteroatome je Molekül.

Wo von "mehr" im Zusammenhang mit funktionell die Rede ist, bedeutet dies, dass die Verbindung mindestens eine, vorzugsweise mindestens zwei oder mehr funktionelle Gruppen trägt.

Als Verbindung der Formel I ist insbesondere Glycerincarbonat zu nennen. Aber auch andere hydroxyfunktionelle (cyclische) Carbonate - hergestellt durch chemische Fixierung von $CO_2$ an Epoxiden und/oder durch Herstellung aus 1,2- und 1,3-Diolen - sind hier zu erwähnen. So können auch Carbonate von Alkoholen wie Trimethylolpropan, Pentaerythrit und von Zuckeralkoholen wie Xylit, Mannit, Erythrit oder Sorbit und/oder Carbonate von hydroxyfunktionellen Glycidylether, wie beispielsweise 1,2- und/oder 1,3-Glycerindiglycidylether oder 2,2-Pentaerythritdiglycidylether, als Verbindung der Formel I verwendet werden. Beispielhaft seien 1,3-Glycerindiglycidylethercarbonat, 5-(Hydroxymethyl)-5-methyl-1,3-dioxan-2-on und/oder 5-(Hydroxy-methyl)-5-ethyl-1,3-dioxan-2-on (TMP-Carbonat) genannt.

Wie bereits in der Einleitung kurz angedeutet sind Verbindungen der Formel I dem Fachmann zugänglich durch Umsetzung von Alkylenoxiden (Epoxiden) als Vorstufen mit (auf diese Weise als $C_1$ fixierbarem und somit der Erdatmosphäre entziehbarem) Kohlendioxid, beispielsweise unter Bedingungen wie in DE 35 29 263 oder DE-OS 26 11 087 und weiter den jeweils darin genannten Dokumenten beschrieben, oder nach analogen Verfahren, oder sie sind kommerziell erhältlich.

Allgemein kann die Herstellung der cyclischen Carbonate insbesondere unter Katalyse, beispielsweise mit basischen Katalysatoren, wie Carbonaten, Bicarbonaten, Alkoholaten, Carboxylaten, Hydroxiden oder Oxiden der Alkali- oder Erdalkalimetalle, oder mit Lewissauren Substanzen, wie zum Beispiel organischen Verbindungen des zwei- oder vierwertigen Zinns oder Titans, zum Beispiel Zinn-(II)-octoat, Zinn-(II)-laureat, Dibutylzinn oder Titantetrabutylat oder mit quartären Ammoniumverbindungen, wie beispielsweise Tetrabutylammoniumbromid, erfolgen, oder nach anderen bekannten Verfahren.

Unter einem Isocyanat mit einer mittleren Funktionalität von 1,5 oder mehr (größer) als 1,5 , beispielsweise von 2,1 bis 5, zum Beispiel von 2,2 bis 4, vorteilhaft z.B. von 2,3 bis 3,5 , ist beispiels-weise ein (Poly-)Isocyanat mit Uretdion-, Isocyanurat-, Iminooxadiazinon-, Uretonimin-, Biuret-, Allophanat- und/oder Carbodiimid-Strukturen (vorteilhaft mit einer Molekulargewichtsverteilung derart, dass keine einzelne Molekülspezies zu mehr als 50 Gewichtsprozent

vorliegt und gleichzeitig mehr als 50 Gewichtsprozent der Ketten aus mindestens 3 + 1 kovalent gebundenen Monomereinheiten/Reaktanten zusammengesetzt sind (siehe genauer Polymerdefinition nach REACH)) oder vorzugsweise eine (z.B. in technischen Herstellprozessen typischerweise anfallende oder nachfolgend gezielt (z.B. durch Zugabe und/oder Abdestillation von Monomeren oder Monomerenmischungen) eingestellte) Mischung von (i) ein oder mehreren monomeren Mono- oder insbesondere Diisocyanaten, wie Diphenylmethandiisocyanat (MDI), insbesondere 4,4'-Diphenylmethyldiisocyanat oder 2,2'-Diphenylmethandiisocyanat oder Mischungen von Diphenylmethandiisocyanat-Isomeren (mit unterschiedlichen Positionen der Isocyanatgruppen an den Phenylkernen) wie den gerade genannten, mit (ii) ein oder mehreren "polymeren" Diphenylmethandiisocyanaten (PMDI), das heißt vorzugsweise Roh-MDI (Rohprodukt der industriellen Herstellung von MDI ohne Trennung der einzelnen Isomeren z.B. durch Destillation) mit (d.h. beinhaltend) mehreren Isomeren und höherfunktionellen Homologen und z.B. einem mittleren Molekulargewicht in der Größenordnung von 200 bis 800 g/mol und einer Funktionalität wie oben angegeben, z.B. mit einem mittleren Molekulargewicht von 280 bis 500, z.B. 310 bis 480 und einer Funktionalität von 2,4 bis 3,4 , z.B. von 3,2. Bevorzugt sind marktübliche PMDI, die aus dem Roh-MDI selbst oder auch aus dem Roh-MDI z.B. durch Abdestillation und/oder Zugabe von monomerem MDI erhalten werden und ein mittleres Molekulargewicht von 310-450 aufweisen und auch Uretdion-, Isocyanurat-, Iminooxadiazinon-, Uretonimin-, Biuret-, Allophanat- und/oder Carbodiimid-Strukturen beinhalten können. Besonders bevorzugt sind marktübliche PMDI mit einer Molekulargewichtsverteilung derart, dass keine einzelne Molekülspezies zu mehr als 50 Gew.-% vorliegt.

Isocyanate mit einer Funktionalität von 1,5 oder größer als 1,5, können beispielsweise auch durch Abmischungen von monomeren (Poly-)Isocyanaten, wie 2,2'- und 4,4'-Diphenylmethandiisocyanat und/oder z.B. p-Toluolsulfonylisocyanat, und höherfunktionellen Polyisocyanaten oder durch Verwendung von polymerem MDI (Roh-MDI), welche höhere Homologe enthalten, erhalten werden.

Unter "Funktionalität" ist die Anzahl der Isocyanatgruppen pro Molekül zu verstehen, bei Diphenylmethandiisocyanat ist diese Funkionalität (im Wesentlichen, d.h. von verunreinigungsbedingten Abweichungen abgesehen) 2, bei den PMDI handelt es sich um eine (in der Regel vom Hersteller angegebene) mittlere Funktionalität, die gemäß der Formel

$$f = \frac{\sum n_i \cdot f_i}{\sum n_i}$$

(f = Funktionalität, $n_i$ = Zahl der Moleküle einer Funktionalität $f_j$,)
errechnet werden kann und vorzugsweise zwischen 2,1 und 5,0 oder in den Bereichen wie oben angegeben liegt.

Die Reaktion zwischen den hydroxyfunktionellen cyclischen Carbonaten (Verbindungen der Formel I) und dem oder den Isocyanaten kann ohne Lösungsmittel oder in Gegenwart eines geeigneten Lösungsmittels (vorhandene(r) Reaktivverdünner können (kann) dann als solches dienen) durchgeführt werden. Vorzugsweise wird die Reaktion in Gegenwart mindestens einem oder mehrerer Reaktivverdünner durchgeführt, insbesondere, soweit sonst zu hohe Viskositäten die Reaktion erschweren würden. "Reaktiv" bezieht sich dabei hier auf die Formulierung des Klebemittels und dessen Aushärtung, nicht auf die Addition des hydroxyfunktionellen cyclischen Carbonates an das Isocyanat.

Die Reaktion kann auch derart geführt werden, dass über eine Vorverlängerung ein Präpolymer gebildet wird und erst danach die noch übrigen Isocyanatgruppen mit dem hydroxyfunktionellen cyclischen Carbonat umgesetzt werden.

Mögliche Reaktivverdünner sind Carbonatharze, Epoxygruppen beinhaltende Reaktivverdünner, (mehr)funktionelle Acrylate und/oder (mehr)funktionelle Acetoactetate. Auch Silane und/oder Siloxane mit oder ohne hydrolysierbaren Restgruppen sind mögliche Reaktivverdünner. Auch können Mischungen von zwei oder mehr der genannten Reaktivverdünner Einsatz finden. Wichtig hierbei ist, dass der mögliche Reaktivverdünner eine gegenüber - aus dem Bereich auch der Epoxidhärtung bekannten (beispielweise) - Aminen bekannte Reaktivität aufweist bzw. gehärtet werden kann. Die Reaktivverdünner können beispielsweise in einem Gewichtsanteil von 0,1 bis 90 Gew.-%, z.B. zwischen 0,5 und 75 Gew.-% oder z.B. zwischen 1 und 60 Gew.-%.

Als (bevorzugte) Epoxygruppen (Epoxidgruppen) beinhaltender Reaktivverdünner (die vorzugsweise nicht in einer Härterkomponente vorliegen sollten, also vorzugsweise (bei einem Zweikomponentensystem nur) in Komponente (A) mit beinhaltet sind), können Glycidylether von aliphatischen, cycloaliphatischen, araliphatischen oder aromatischen Mono- oder insbesondere Polyalkoholen Verwendung finden, wie Monoglycidylether, z.B. o-Kresylglycidylether, und/oder Glycidylether mit einer Epoxy-Funktionalität von mindestens 2, wie 1,4-Butandioldiglycidylether, Cyclohexandimethanoldiglycidylether, Hexandioldiglycidylether und/oder Tri- oder höhere Glycidylether, z.B. Glycerintriglycidylether, Pentaerythrittetraglycidylether oder Trimethylolpropantriglycidylether, oder auch Mischungen von zwei oder mehr dieser Epoxygruppen beinhaltender Reaktivverdünner auch unterschiedlicher Funktionalität.

Als Reaktivverdünner aus dem Bereich der niedrigviskosen Carbonatharze können z.B. die durch chemische Fixierung von $CO_2$ erhältlichen bzw. erhaltenen cyclischen Carbonate aus den oben genannten Epoxygruppen beinhaltenden Reaktivverdünnern Verwendung finden. Beispielhaft erwähnt seien hier: 1,4-Butandiolglycidylethercarbonat, Cyclohexandimethanoldiglycidylethercarbonat, Hexandioldiglycidylethercarbonat und/oder Tri- oder höhere Glycidylethercarbonate, wie z.B. Glycerintriglycidylethercarbonat, Pentaerythrittetraglycidylethercarbonat oder Trimethylolpropantriglycidylethercarbonat. Mischungen von zwei oder mehr dieser Reaktivverdünner aus dem Bereich der niedrigviskosen Carbonatharzen, auch unterschiedlicher Funktionalität, sind ebenfalls möglich.

Als (mehr)funktionelle Acrylat-Reaktiwerdünner sind insbesondere Acrylat- oder Acrylamidmonomere, wie Acrylsäure oder vorzugsweise deren Ester (als Acrylate bezeichnet) oder Amide, insbesondere Acrylate, insbesondere der Formel (H)-C(=$CH_2$)-C(=O)-OX, worin X ein gegebenenfalls substituierter oder mehrfach substituierter Alkylrest mit 1 bis 12 Kohlenstoffatomen ist, wie Mono-, Di-, Tri-, oder Polyacrylate (einschließlich Hydroxyalkylacrylaten, wie Hydroxypropylacrylat oder Hydroxyethylacrylat), Alkylacrylate mit 1 bis 10 Acrylatgruppen, wie Mono-, Di-, Tri-, Tetra-, Penta-, Hexa- oder polyacrylate, z.B. Alkyldi- oder triacrylate, wie 1,2-Ethandioldiacrylat wie 1,3- oder insbesondere 1,4-Butandioldiacrylat, Hexandioldiacrylat, Diethylenglykoldiacrylat, Trimethylolpropantriacrylat, Glycerintriacrylat, Polyglycerinpolyacrylat, Polyethylenglykoldiacrylat, Cycloalkyl-, Bicycloalkyl- oder Heterocycloalkylacrylate, worin Cycloalkyl oder Bicycloalkyl 5 bis 7 Ringkohlenstoffatome aufweist und Heterocyclyl 5 oder 6 Ringatome hat und 1 oder 2 Ringheteroatome ausgewählt aus N, O und S aufweist, wie Tetrahydrofurfurylacrylat oder Isobornylacrylat, oder Acetoacetoxyalkylacrylat. Weitere mögliche (mehr)funktionelle Acrylat-Reaktiwerdünner sind Ester der Acrylsäure mit (jeweils propoxyliertem oder ethoxyliertem und/oder unterschiedliche Propoxylierungs- oder Ethoxylierungsgrade aufweisendem) aromatischem Diol-, wie Bisphenol-A-, Bisphenol-F- oder Novolaken, Epoxyacrylate (insbesondere in Form von Umsetzungsprodukten von Di- oder Polyepoxiden, z.B. Bisphenol-A-, Bisphenol-F- oder Novolak-di- und/oder -polyglycidylethern, mit Acrylsäure, Urethan- und/oder Harnstoffacrylate (was, wie dem Fachmann bekannt, auch vorverlängerte und/oder oligomere Urethan- und/oder Harnstoffacrylate umfasst.

Reaktivverdünner auf Basis einer Acetoacetato-Verbindung (Acetoacetate) sind beispielsweise Acetylaceton, Acetoacetatoethylacrylat, Triacetoacetatotrimethylolpropan und/oder Cyanoacetate.

Bei den als reaktivverdünnend wirkenden Silane und/oder Siloxane mit oder ohne hydrolysierbaren Restgruppen handelt es sich vorzugsweise um mit Epoxygruppen funktionalisierte Silane und/oder Siloxane und/oder Siloxanooligomere. Beispiele für solche Silane sind: 3-Glycidoxypropyltrimethoxysilan, 3-Glycidoxypropyltriethoxysilan, 3-Glycidoxypropylmethyldimethoxysilan und 3-Glycidoxypropylmethyldiethoxysilan und/oder aus den genannten Silanen erhältliche bzw. erhaltene Siloxane und/oder Siloxanoligomere. Auch hier sind Mischungen von zwei oder mehr davon möglich.

Für die Herstellung des oben genannten bzw. möglichen Präpolymeren finden dabei zum Erzielen einer mittleren Isocyanat-Funktionalität von 1,5 oder größer als 1,5 die oben genannten Isocyanate und (Poly-)Alkohole mit einer oder zwei oder mehr Hydroxygruppen pro Molekül und/oder (Poly-)Amine mit einer oder mehr Aminogruppen pro Molekül Verwendung, oder es werden Isocyanate mit einer Funktionalität von 2 mit (Poly-)Alkoholen, (Poly-)Aminen oder Aminolen mit einer mittleren OH- und/oder Amino-Funktionalität von mehr als 1, beispielsweise 2, eingesetzt.

(Poly-)Alkohole (Mono-, Di- oder höherfunktionale Alkohole) sind dabei insbesondere mono-, di- oder höherfunktionale Alkohole, z.B. Ethanol, Propanol, Hydroxyethylacrylat und/oder Folgeprodukte des Ethylen- oder Propylenoxids, wie Ethandiol, Di- bzw. Triethylenglykol, Propan-1,2- oder -1-3-diol, Dipropylenglykol, andere Diole, wie 1,2-, 1,3- oder 1,4-Butandiol, 1,6-Hexandiol, Neopentylglykol, 2-Ethylpropan-1,3-diol oder 2,2-Bis(4-hydroxycyclohexyl)-propan, Triethanolamin, Bisphenol A oder Bisphenol F oder deren Oxyethylierungs-, Hydrierungs- und/oder Halogenierungsprodukte, höherwertige Alkohole, wie z.B. Glycerin, Trimethylolpropan, Hexantriol und Pentaerythrit, hydroxylgruppenhaltige Polyether, z.B. Oligomere aliphatischer oder aromatischer Oxirane und/ oder höherer cyclischer Ether, z.B. Ethylenoxid, Propylenoxid, Styroloxid und Furan, Polyether mit jeweils endständigem Hydroxy, die in der Hauptkette aromatische Struktureinheiten enthalten, z.B. die des Bisphenol A bzw. F, hydroxylgruppenhaltige Polyester auf der Basis der oben genannten Alkohole bzw. Polyether und Dicarbonsäuren bzw. ihrer Anhydride, z.B. Adipinsäure, Phthalsäure, Isophthalsäure, Terephthalsäure, Tetra- bzw. Hexahydrophthalsäure, Endomethylentetrahydrophthalsäure, Tetrachlorphthalsäure oder Hexachlor-endomethylentetrahydrophthalsäure, Maleinsäure, Fumarsäure, Itaconsäure, Sebacinsäure oder dergleichen. Besonders bevorzugt sind Hydroxylverbindungen mit Kettenversteifung bewirkenden aromatischen Struktureinheiten, Hydroxyverbindungen mit ungesättigten Komponenten zur Erhöhung der Vernetzungsdichte, wie Fumarsäure, oder verzweigte oder sternförmige Hydroxyverbindungen, insbesondere drei-bzw. höherfunktionale Alkohole und/ oder Polyether bzw. Polyester, die deren Struktureinheiten enthalten. Besonders bevorzugt sind Niederalkandiole (ergeben divalente Reste -O-Niederalkylen-O-).

Aminole (Aminoalkohole) sind Verbindungen, die insbesondere eine oder mehrere Hydroxy- und eine oder mehrere Aminogruppen in ein und demselben Molekül enthalten. Bevorzugte Beispiele sind aliphatische Aminole, insbesondere Hydroxyniederalkylamine (ergeben Reste -NH-Niederalkylen-O- oder -O-Niederalkylen-NH-), wie Ethanolamin, Diethanolamin oder 3-Aminopropanol, oder aromatische Aminole, wie 2-, 3- oder 4-Aminophenol.

(Poly-)Amine (Mono-, Di- oder höherfunktionale Amine) sind organische Aminoverbindungen mit 1 oder mehr

Aminogruppen, insbesondere wie Aminosäuren, z.B. Glycin oder difunktionale Amine, wie Hydrazin, N,N'-Dimethylhydrazin, aliphatische Di- oder Polyamine, insbesondere Niederalkandiamine (ergeben Reste -NH-Niederalkyl-NH-), wie Ethylendiamin, 1,3-Diaminopropan, Tetra- oder Hexamethylendiamin oder Diethylentriamin, oder aromatische Di- oder Polyamine, wie Phenylendiamin, 2,4- und 2,6-Toluoldiamin, Benzidin, o-Chlorbenzidin, 2,5-p-Dichlorphenylendiamin, 3,3'-Dichlor-4,4'-diamino-diphenylmethan oder 4,4'-Diaminodiphenylmethan, Polyetherdiamine (Polyethylenoxide mit endständigen Aminogruppen) oder Polyphenyl/Polymethylen-polyamine, die durch Kondensation von Anilinen mit Formaldehyd erhältlich sind.

Das Verhältnis von freien Isocyanatgruppen des oder der Isocyanate zu Hydroxygruppen der cyclischen Carbonate (Verbindungen der Formel I) wird vorteilhaft derart aufeinander abgestimmt, dass eine schnelle und vollständige Umsetzung der Isocyanatgruppen resultiert und im Produkt praktisch keine freien Isocyanatgruppen mehr vorhanden sind, was beispielsweise mittels IR-Spektroskopie nachgewiesen werden kann. D.h., die Mol-Menge der Hydroxygruppen (und damit die korrelierende Mol-Menge an hydroxfunktionellem cyclischen Carbonat) ist vorzugsweise größer als die Mol-Menge der Isocyanatgruppen, z.B. 1,03 bis 5 mal so groß, wie z.B. 1,05 bis 4 mal so groß oder 1,1 bis 3 mal so groß. Überschüssiges hydroxyfunktionelles cyclisches Carbonat dient als Reaktivverdünner.

Die Reaktion der Isocyanate und den Verbindungen der Formel I findet vorzugsweise bei Temperaturen im Bereich von 0 bis 100 °C, z.B. zwischen Raumtemperatur (23 °C) und 85 °C, statt.

Das Verfahren zur Herstellung von cyclischen Carbonatharzen, insbesondere von Carbonat-Urethan-Harzen, kann in Gegenwart von einem Katalysator, wobei entsprechende Katalysatoren, die die Reaktion zwischen Hydroxygruppen und Isocyanatgruppen katalysieren, dem Fachmann hinreichend bekannt sind, beispielsweise einem tertiären Amin, wie 1,2-Dimethylimidazol, Diazabicyclooctan, Diazabicyclononan, oder vorzugsweise einer Organometallverbindung (z.B. von Sn, Pb, Bi, Al, K wie Kaliumacetat und auch von Übergangsmetallen wie Ti, Zr, Fe, Zn, Cu); sowie Mischungen von zwei oder mehr davon; beispielsweise (bezogen auf die Reaktionsmischung) in einem Anteil von 0,001 bis 2,5 Gew.-%, stattfinden.

Beispiele für geeignete Katalysatoren sind dem Fachmann bekannt, beispielsweise wie aus "Polyurethane Kunststoff-Handbuch 7" von Becker, G.W.; Braun, D.; Oertel, G.; 3. Auflage, Carl Hanser Verlag, 1993, ersichtlich.

Der Härter (ggf. als Härterkomponente, z.B. Komponente B wie unten im Zusammenhang beschrieben) beinhaltet mindestens eine zur Epoxidhärtung (Epoxid stellvertretend hier auch die cyclischen Carbonatgruppen der cyclischen Carbonatharze umfassend) gebräuchliche Verbindung (Reaktionspartner bei der Polyaddition). Der Begriff "Härter" bedeutet dabei vorzugsweise mindestens eine zur Epoxidhärtung gebräuchliche Verbindung (als eigentlicher "Härter" im engeren Sinne) mit oder ohne ein oder mehrere weitere Zusätze, beispielsweise wie nachfolgend definiert, mit anderen Worten, die komplette Härterkomponente. Der Härter kann als separate Komponente und/oder (insbesondere in geschützter Form, d.h. z.B. in mikroverkapselter Form) auch in der Reaktionsharzformulierung (als einer härtbaren Komponente, d.h. einer solchen, die nach Mischung mit dem Härter nach Aufbrechen der Hülle der Mikrokapsel durch Polymerisation aushärtet) eingearbeitet sein. Übliche Zusätze können zugesetzt sein, wie z.B. Füllstoffe (insbesondere wie unten definiert) und/oder (insbesondere zur Herstellung einer Paste oder Emulsion) Lösungsmittel, wie Benzylalkohol und/oder Wasser. Die weiteren Zusätze der Härterkomponente eines erfindungsgemäßen oder erfindungsgemäß verwendeten Kunstharz-Befestigungssystems können beispielsweise in einem Gewichtsanteil von insgesamt 0,01 bis 70 Gew.-%, z.B. von 1 bis 40 Gew.-% bezogen auf die Härterkomponente vorgesehen sein.

Bei den zur Epoxidhärtung gebräuchlichen Verbindungen (die als Reaktionspartner bei der Polyaddition fungieren) handelt es sich insbesondere um solche mit zwei oder mehr Gruppen ausgewählt aus Amino, Imino, und Mercapto, beispielsweise entsprechende Amine (bevorzugt), Thiole, oder Aminothiole, oder Gemische davon, beispielsweise wie in Lee H and Neville K, "Handbook of Epoxy Resins" (New. York: McGraw-Hill), 1982, genannt, welches hier diesbezüglich durch Bezugnahme aufgenommen wird, beispielsweise darin genannte Di- oder Polyamine, und/oder Di- oder Polythiole.

Die zur Epoxidhärtung (allgemein) gebräuchlichen Verbindungen umfassen beispielsweise in einer Ausführungsform der Erfindung Di- oder Polyamine und/oder Di- oder Polythiole. Die einsetzbaren Di- oder Polyamine und/oder Di- oder Polythiole können sowohl linear als auch verzweigt sein. Das Molekülgerüst der Di- oder Polyamine und/oder der Di- oder Polythiole kann aliphatische, aromatische, aliphatisch-aromatische, cycloaliphatische und heterocyclische Strukturen enthalten. Im Molekül können primäre und/oder sekundäre und tertiäre Amine und/oder primäre Thiole vorhanden sein, jedoch müssen mindestens zwei (-NHR-)-bzw. zwei (HSR-)-Atomgruppierungen, bevorzugt zwei Aminogruppen und/oder Thiolgruppen, enthalten sein. Die Amin- bzw. Thiol-Funktionen selbst sind aliphatisch, d.h. dass die dem Aminstickstoff bzw. die dem Thiolschwefel unmittelbar benachbarten Kohlenstoffatome nicht Teil einer aromatischen Ringstruktur sind.

Die erfindungsgemäß verwendeten bzw. verwendbaren Di- oder Polyamine und/oder Di- oder Polythiole werden als Einzelverbindung oder auch als Mischung der entsprechenden, als Di- oder Polyamine und/oder Di- oder Polythiole verwendbaren Verbindungen eingesetzt.

Die Di- oder Polyamine werden bevorzugt aus der Gruppe der Alkylendiamine und/oder der Cycloalkylendiamine ausgewählt.

Unter Alkylendiaminen sind Verbindungen der allgemeinen Formel $R^aR^bN\text{-}E\text{-}NR^cR^d$ zu verstehen, bei denen

$R^a$, $R^b$, $R^c$, $R^d$ unabhängig voneinander H, Alkyl- oder Cycloalkylreste sein können. E bedeutet eine lineare oder verzweigte, gesättigte oder ungesättigte Alkylenkette mit ≥ 2 C-Atomen. Bevorzugte Beispiele sind Diaminoethan, Diaminopropan und weitere Homologe wie 1,4-Diaminobutan, 1,5-Diaminopentan und 1,6-Diaminohexan. Diese drei zuletzt genannten Verbindungen kommen zudem in der Natur vor, so dass bei deren Verwendung zusätzlich Produkte mit einem erhöhten Bio-C-Gehalt entstehen. Weitere Beispiele für Alkylendiamine sind Isophorondiamin, Dioxadecandiamin und N,N-Bis(3-Aminopropyl)-dodecylamin.

Unter Cycloalkylendiaminen sind Verbindungen der allgemeinen Formel $R^eR^fN\text{-}G\text{-}NR^gR^h$ zu verstehen, bei denen $R^e$, $R^f$, $R^g$, $R^h$ unabhängig voneinander H, Alkyl- oder Cycloalkylreste sein können. G bedeutet einen gesättigten oder ungesättigten Cycloalkylrest mit ≥ 3 C-Atomen, vorzugsweise ≥ 4 C-Atomen. Bevorzugt sind Diaminocyclopentane, Diaminocyclohexane, Diaminocycloheptane, beispielsweise 1,4-Cyclohexandiamin; 4,4'-Methylenbis-cyclohexylamin; 4,4'-Isopropylen-bis-cyclohexylamin, Isophorondiamin, m-Xylylendiamin, N-Aminoethylpiperazin oder Gemische daraus.

Die Diamine können auch sowohl Alkyl- als auch Cycloalkylreste gemeinsam enthalten. Bevorzugte Beispiele sind Aminoethylpiperazin, 1,8-Diamino-p-menthan, Isophorondiamin, 1,2-(Bisaminomethyl)-cyclohexan, 1,3-(Bisaminomethyl)-cyclohexan, 1,4-(Bisaminoethyl)-cyclohexan, Bis-(4-aminocyclohexyl)-methan.

Bevorzugt können auch polyfunktionelle Amine eingesetzt werden. Insbesondere sind dies aminfunktionalisierte Polyalkylenglykole, wie 1,2-Bis-(aminoethoxy)-ethan; 1,13-Diamino-4,7,10-trioxatridecan. Diese aminfunktionalisierte Polyalkylenglykole sind kommerziell beispielsweise als Jeffamine über die Firma Huntsman Corp. erhältlich.

Ebenfalls bevorzugte einsetzbare polyfunktionelle Amine sind Verbindungen der allgemeinen Formel $H_2N\text{-}(CH_2)_i\text{-}NH\text{-}[(CH_2)_j\text{-}NH]_k\text{-}(CH_2)_l\text{-}NH_2$, worin i, j und l unabhängig voneinander für 2 bis 4 stehen und k für 0, 1, 2, 3 oder 4 steht, wie z.B. Diethylentriamin, Triethylentetramin, Tetraethylenpentamin und weitere Homologe, Dipropylentriamin, Bis-(3-aminopropyl)-amin und ähnliche.

Weiterhin bevorzugt können die Di- oder Polyamine aus der Gruppe der Polyimine, Aminoamide, Polyaminoamide, Mannich-Basen und der Aminaddukte (Bucherer-Addukte und Michael-Additions-Addukte) ausgewählt sein.

Bevorzugte Polyimine sind Polyethylenimine. Die Aminwasserstofffunktionen der Polyethylenimine können auch teilweise modifiziert sein, wie zum Beispiel durch Alkylierung, vorzugsweise Ethoxylierung und/oder Propoxylierung. Bevorzugt einsetzbare Polyethylenimine sind kommerziell von der Firma BASF unter dem Handelsnamen Lupasol erhältlich. Polyaminoamide sowie Aminoamide enthalten sowohl Amin- als auch Amid-Funktionalitäten. Polyaminoamide werden durch Polykondensation von Polyaminen und Dicarbonsäuren hergestellt. Entsprechende Polyaminoamide sind in Lee H and Neville K, "Handbook of Epoxy Resins" (New. York: McGraw-Hill), 1982, beschrieben.

Bevorzugte Aminoamide sind Monomere mit einer Aminfunktionalität von 2 oder mehr, besonders bevorzugt haben die Aminoamide eine Funktionalität von mehr als 2 und sind aus nachwachsenden Rohstoffen zugänglich. Derartige Produkte lassen sich zum Beispiel durch Umsetzung natürlich vorkommender Ester mit mehr als 2 Estergruppen mit Diaminen erhalten. Beispielhaft und stellvertretend sei hier auf trifunktionelle Aminoamide ausgehend von Zitronensäureestern, hergestellt gemäß der DE 10 104 437, verwiesen. Prinzipiell können beliebige Diamine (beispielsweise die vorgenannten Di- oder Polyamine) eingesetzt werden. Ebenfalls können anstatt Zitronensäure bzw. Zitronensäureester auch andere natürlich vorkommende Säuren mit einer entsprechenden Zahl an funktionellen Säuregruppen verwendet werden. Entsprechende Produkte sind dem Fachmann bekannt.

Mannich-Basen können insbesondere wie in der Druckschrift WO 2005/090433, vor allem auf den Seiten 3, letzter, bis S. 6, 2. Absatz offenbart, wie in Beispiel 1 oder insbesondere 2 davon, oder wie in der Druckschrift EP 0 645 408 und der bisher noch nicht veröffentlichten deutschen Patentanmeldung DE 10 2013 113 465.3 offenbart, die hier diesbezüglich durch Bezugnahme aufgenommen werden, alleine oder im Gemisch mit ein oder mehreren weiteren Di- oder Polyaminen eingesetzt werden.

Als Amin-Addukte kommen insbesondere Bucherer-Addukte wie in der Druckschrift EP 0 824 124 offenbart und Michael-Addukte, die durch Reaktion von Acrylsäureestern mit geeigneten Vertretern der oben erwähnten Di- oder Polyamine, in Betracht.

Die Di- oder Polythiole werden bevorzugt aus der Gruppe der ethoxylierten und/oder propoxylierten Alkohole aus Mono-, Di-, Tri-, Tetra-, Pentaolen und/oder anderen Polyolen mit Thiolendgruppen (z.B. Capcure 3-800 der Firma Cognis) und/oder aus der Gruppe der Estergruppen beinhaltenden Thiolen ausgewählt. Beispielsweise kann es sich um Ester von $\alpha$-Mercaptoacetat oder $\beta$-Mercaptopropionat mit Diolen, Triolen, Tetraolen, Pentaolen oder anderen Polyolen handeln.

Es können auch Mischungen von zwei oder mehr der genannten zur Epoxidhärtung gebräuchlichen Verbindungen verwendet werden bzw. beinhaltet sein.

Die zur Epoxidhärtung gebräuchlichen Verbindungen liegen in den Ausführungsformen der Erfindung vorzugsweise in Mengen von bis zu 95 Gew.-%, vorzugsweise von 2 bis 70 Gew.-%, beispielsweise von 10 bis 50 % vor.

Bezogen auf die Härterkomponente liegt der Anteil dieser Verbindungen in einer möglichen bevorzugten Ausführungsform der Erfindung bei 1 bis 100 Gew.-%, beispielsweise bei 3 bis 95 Gew.-%, z.B. 4 bis 95 Gew.-%, 5 bis 90 Gew.-% oder 10 bis 80 Gew.-%.

In einer weiteren Ausführungsform der Erfindung werden die cyclischen Carbonatharze verwendet zur Herstellung von diese beinhaltenden (vorzugsweise mehr-, insbesondere zwei-komponentigen) Klebemitteln zur Befestigung von Verankerungsmitteln in Löchern, welche dadurch gekennzeichnet ist, dass ein erfindungsgemäß verwendbares cyclisches Carbonatharz, insbesondere ein cyclisches Carbonat-Urethan-Harz, als Harzkomponente mit weiteren Inhaltsstoffen formuliert (gemischt, gegebenenfalls unter Trennung von Harzkomponente und Härterkomponente) wird, die üblicherweise in Klebemitteln wie z.B. auf Vinylesterure-than- und/oder Epoxidbasis - für das Befestigen von Verankerungselementen in Löchern - Einsatz finden.

Die so erhältlichen Kunstharz-Befestigungssysteme umfassend ein nach dem offenbarten Verfahren erhältliches cyclisches Carbonatharz, insbesondere ein cyclisches Carbonat-Urethan-Harz (CU-Harz), sind, ebenfalls Gegenstand der vorliegenden Offenbarung.

In einer besonderen Ausführungsform werden dann unter Fortsetzung dieser Verwendung die so erhältlichen erfindungsgemäßen oder erfindungsgemäß zu verwendenden Klebemittel zum Befestigen von Verankerungsmitteln in Löchern oder Spalten, insbesondere in Bohrlöchern, in Gebäudesubstraten verwendet.

Wichtige Beispiele für weitere Inhaltsstoffe in Kunstharz-Befestigungssystemen gemäß der Erfindung bzw. für die erfindungsgemäße Verwendung sind hier Beschleuniger, Inhibitoren, nicht reaktive Verdünner, reaktive Verdünner, Thixotropiermittel, Füllstoffe und/oder weitere Additive, oder Mischungen von zwei oder mehr dieser Inhaltsstoffe.

Als Beschleuniger können z.B. tert.-Amine, wie Imidazole oder tert.-Aminophenole, wie Tris-2,4,6-dimethylaminomethylphenol, Organophosphine oder Lewis-Basen oder -Säuren, wie Phosphorsäureester, oder Gemische von zwei oder mehr davon, in einer oder (insbesondere bei Mehrkomponentensystemen) bei mehreren der Komponenten, bevorzugt jeweils in einer Härterkomponente, beinhaltet sein. Weitere mögliche Beschleuniger sind Basen, deren konjugierte Säure einen pKa von 13 oder mehr besitzt. Es sei hier auf die DE 698 27 788 T2 verwiesen, die hier diesbezüglich durch Bezugnahme aufgenommen wird. Bevorzugte Basen sind t-Butoxid und N,N-Bis(Trimethylsilyl)-amid(Anion), wobei t-Butoxid besonders bevorzugt ist. Es ist auch bevorzugt, dass die Base in Form eines Alkalimetall oder Ammoniumsalzes zugegeben wird und stärker bevorzugt, wenn es ein Kaliumsalz ist. Die Beschleuniger haben, vorzugsweise einen Anteil (Konzentration) von 0,005 bis 10, insbesondere von 0,1 bis 5 Gew.-%.

Als Inhibitoren bzw. Verzögerer können beispielsweise organische und/oder anorganische Säuren zugesetzt werden, welche aufgrund der eintretenden Protonierung des freien Elektronenpaares am Stickstoff bzw. am Schwefel dessen Aktivität herabsetzen. Bevorzugt werden die Säuren der Härterkomponente zugesetzt. Die Inhibitoren haben vorzugsweise einen Anteil von bis zu 1 Gew.-%, insbesondere falls vorhanden zwischen 0,0001 und 0,5 Gew.-%, z.B. zwischen 0,01 und 0,1 Gew.-%.

Als nicht reaktive Verdünner können beispielsweise Pflanzenöle, wie Rizinusöl, oder ferner Bioalkohole und Fettsäuren und deren Ester zugesetzt werden, oder Gemische von zwei oder mehr davon, beispielsweise in einem Anteil von 3 bis 60 Gew.-%, z.B. von 4 bis 55 Gew.-%.

Als Thixotropiermittel können übliche thixotropieverursachende Rheologiehilfsmittel verwendet werden, wie pyrogene Kieselsäure oder (z.B. mit Silanen) oberflächenbehandelte Kieselsäure. Sie können z.B. in einem Gewichtsanteil von 0,01 bis 50 Gew.-%, beispielsweise von 0,5 bis 20 Gew.-%, zugesetzt werden.

Als Füllstoffe finden übliche Füllstoffe, insbesondere werden unten noch einmal erwähnt, Kreiden, Quarzsand, Quarzmehl, Korund oder dergleichen, die als feinteilige Pulver in körniger Form oder in Form von Formkörpern zugesetzt sein können, Verwendung, oder andere, oder Gemische davon, wobei die Füllstoffe ferner oder insbesondere auch silanisiert sein können. Die Füllstoffe können in einer oder in mehreren Komponenten eines erfindungsgemäßen bzw. erfindungsgemäß zu verwendenden mehrkomponentigen Kunstharz-Verklebungsmittels, beispielsweise einer oder beiden Komponenten eines entsprechenden Zweikomponentenkits, vorhanden sein; der Anteil an Füllstoffen beträgt vorzugsweise 0 bis 90 Gew.-%, beispielsweise 10 bis 50 Gew.-% (wobei auch beim Einbringen von Verankerungselementen zerstörtes Hüllmaterial (z.B. zersplittertes Glas oder zersplitterter Kunststoff), beispielsweise Scherben aus Patronen, mit als Füllstoff gerechnet werden kann). Zusätzlich oder alternativ zu ein oder mehreren der genannten Füllstoffe können hydraulisch härtbare bzw. härtende Füllstoffe, wie Gips, Branntkalk oder Zement (z.B. Tonerd- oder Portlandzement), Wassergläser oder aktive Aluminiumhydroxide, oder zwei oder mehr davon, zugesetzt werden.

Auch weitere Additive können zugesetzt sein, wie Weichmacher, nicht reaktive Verdünnungsmittel, Flexibilisatoren, Stabilisatoren, Rheologiehilfsmittel, Netz- und Dispergiermittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, Gemische von zwei oder mehr davon. Derartige weitere Zusätze können vorzugsweise insgesamt in Gewichtsanteilen von insgesamt 0 bis 90 %, beispielsweise von 0 bis 40 Gew.-%, zugesetzt sein.

"Auf Basis" bedeutet, dass die erfindungsgemäßen oder erfindungsgemäß zu verwendenden Kunstharz-Befestigungssysteme oder deren Komponenten neben den genannten Bestandteilen auch weitere übliche Bestandteile bzw. Inhaltsstoffe (z.B. Additive oder andere wie oben oder unten genannte Bestandteile) beinhalten können. Diese weiteren Inhaltsstoffe können zusammen beispielsweise in einer Menge von insgesamt bis zu 80, vorzugsweise zwischen 0,01 und 75 Gew.-%, vorliegen. Auch wo nicht ausdrücklich "auf Basis" erwähnt wird, sind derartige übliche Bestandteile bzw. Inhaltsstoffe mit beinhaltet.

Unter einem Loch oder Spalt ist ein solches Loch oder ein solcher Spalt zu verstehen, das oder der in einem festen (insbesondere bereits als solcher fertiggestellten) Untergrund (Gebäudesubstrat), insbesondere Mauerwerk oder Beton, ggf. auch in einem rissigen Substrat, wie rissigem Beton, vorhanden und von mindestens einer Seite her zugänglich ist, beispielsweise ein Bohrloch, oder ferner ein beim Mörteln mit anorganischen Mörtel- oder Putzmassen (wie mit Zement oder Gips) ausgesparter Bereich oder dergleichen.

In einer besonderen Ausführungsform der Erfindung sind die härtbaren Komponenten und die zugehörigen Härter (Härterkomponenten) voneinander getrennt in einem Zwei- oder Mehrkomponentensystem aufbewahrt, bevor sie (im Rahmen der erfindungsgemäßen Verwendung) am gewünschten Ort (z.B. bei oder in einem Loch oder Spalt, wie Bohrloch) miteinander vermischt werden.

Die erfindungsgemäßen oder erfindungsgemäß zu verwendenden Kunstharz-Befestigungssysteme können so beispielsweise als Mehr-Komponentensysteme (z.B. Mehrkomponentenkit) vorgesehen sein und werden auch als solche verwendet.

Unter einem Mehrkomponentenkit ist insbesondere ein Zwei- oder (ferner) Mehrkomponentenkit (vorzugsweise ein Zweikomponentenkit) mit einer Komponente (A), welche ein cyclisches Carbonatharz wie vor- und nachstehend beschrieben, beinhaltet, und mit einer Komponente (B), die mindestens jeweils einen zugehörigen Härter wie oben und nachfolgend definiert, beinhaltet, wobei weitere Zusätze in einer oder beider der Komponenten vorgesehen sein können, vorzugsweise eine Zwei- oder ferner Mehrkammervorrichtung, zu verstehen, worin die miteinander reaktionsfähigen Komponenten (A) und (B) und ggf. weitere separate Komponenten so enthalten sind, dass ihre Bestandteile während der Lagerung nicht (insbesondere unter Aushärtung) miteinander reagieren können, vorzugsweise so, dass ihre Bestandteile vor der Anwendung nicht miteinander in Berührung kommen, das es jedoch ermöglicht, die Komponenten (A) und (B) und gegebenenfalls weitere Komponenten zur Befestigung an der gewünschten Stelle, beispielsweise direkt vor oder in einem Loch oder Spalt, so zu vermischen und erforderlichenfalls einzubringen, dass dort die Härtungsreaktion stattfinden kann. Auch geeignet sind Patronen, beispielsweise aus Kunststoff, Keramik oder insbesondere Glas, in denen die Komponenten durch (beispielsweise bei Eintreiben eines Verankerungselements in ein Loch oder einen Spalt, wie ein Bohrloch) zerstörbare Abgrenzungswandungen oder integrierte separate zerstörbare Behältnisse voneinander getrennt angeordnet sind, beispielsweise als ineinander verschachtelte Patronen, wie Ampullen; sowie insbesondere Mehr- oder vorzugsweise Zweikomponenten¬kar¬tuschen (die ebenfalls besonders bevorzugt sind), in deren Kammern die mehreren oder vorzugsweise zwei Komponenten (insbesondere (A) und (B)) des erfindungsgemäßen oder erfindungsgemäß zu verwendenden Kunstharz-Befestigungssystems mit oben und nachstehend genannten Zusammensetzungen zur Aufbewahrung vor der Nutzung enthalten sind, wobei vorzugsweise auch ein Statikmischer zum entsprechenden Kit gehört.

Vorzugsweise liegt das Volumenverhältnis der Komponente (A) zur Komponente (B) im Bereich von 20 : 1 bis 1 : 1, insbesondere im Bereich von 12 : 1 bis 2 : 1, z.B. bei 10 : 1 bis 3 : 1.

Vorteilhaft können die Verpackungsmaterialien (wie Folien, Kartuschen (auch Statikmischer) oder Kunststoffpatronen) ebenfalls aus Kunststoffen mit hohem oder vollständigem biogenem Kohlenstoffanteil ausgeführt sein, beispielsweise aus entsprechenden Polyamiden oder dergleichen.

Die Verwendung eines erfindungsgemäß zu verwendenden oder erfindungsgemäßen Kunstharz-Befestigungssystems am gewünschten Einsatzort erfolgt durch Mischen der zugehörigen (vor Mischung reaktionshemmend separierten) Komponenten, insbesondere nahe bei und/oder direkt vor einem Loch oder (beispielsweise insbesondere bei Verwendung von Kartuschen mit Statikmischern) direkt vor und/oder (insbesondere beim Zerstören entsprechender Patronen oder Ampullen) innerhalb eines Loches oder Spaltes, z.B. einem Bohrloch.

Unter "Einmörteln" ist insbesondere eine (stoff- und/oder formschlüssige) Befestigung von Verankerungsmitteln aus Metall (z.B. Hinterschneidanker, Gewindestangen, Schrauben, Bohranker, Bolzen) oder ferner aus einem anderen Material, wie Kunststoff oder Holz, in festen (vorzugsweise bereits als solche fertiggestellten) Gebäudesubstraten, wie Beton oder Mauer-werk, insbesondere, soweit sie Bestandteile von künstlich errichteten Bauwerken sind, vor allem Mauerwerk, Decken, Wände, Böden, Platten, Pfeilern oder dergleichen (z.B. aus Beton, Naturstein, Mauerwerk aus Vollsteinen oder Lochsteinen, ferner Kunststoff oder Holz), insbesondere in Löchern, wie Bohrlöchern, zu verstehen. Mittels dieser Verankerungsmittel können dann beispielsweise Geländer, Abdeckungselemente, wie Platten, Fassadenelemente oder andere Bauelemente befestigt werden.

Wo von "Gemischen von zwei oder mehr davon" die Rede ist, beinhaltet dies insbesondere Gemische von mindesten einem der jeweils genannten Bestandteile, die als bevorzugt hervorgehoben sind, mit ein oder mehreren anderen, insbesondere ein oder mehreren ebenfalls als bevorzugt gekennzeichneten Bestandteilen bzw. Inhaltsstoffen.

"Als solche fertiggestellt" bedeutet insbesondere, dass die Substrate bis auf mögliche Oberflächenmodifikationen (wie Beschichtung, z.B. Verputzen oder Lackieren) oder dergleichen bereits fertiggestellt (z.B. als Bausteine oder Mauern) und nicht erst gleichzeitig mit dem Verklebungsmittel (Klebemittel) fertiggestellt werden oder aus diesem bestehen. Mit anderen Worten: Das Verklebungsmittel ist dann nicht selbst bereits fertiggestelltes Substrat.

Spezifische Ausführungsformen der Erfindung betreffen auch die in den Ansprüchen und der Zusammenfassung aufgeführten Varianten, soweit sie unter die Ansprüche fallen.

Die nachfolgenden Beispiele dienen der Illustration der Erfindung, ohne ihren Umfang einzuschränken:

Beispiel 1: Allgemeine Arbeitsvorschrift I: Synthese der cylischen Carbonatharze am Beispiel des 4,4'-Diphenylmethandiurethandiglycerincarbonates mit Trimethylolpropantriglycidylether als Reaktivverdünner:

In einem 250 mL Glaskolben mit Rückflusskühler mit Trockenrohr, Rührer, Tropftrichter und Thermometer werden - in den in Tab. 1 (Beispiel 3) genannten Mengen - Trimethylolpropantriglycidylether und Glycerincarbonat vorgelegt und im Ölbad auf 60 °C aufgeheizt. Das "PMDI" wird so langsam zur Reaktionsmischung zugetropft, dass die Temperatur nicht über 80 °C ansteigt. Nach vollständiger Zugabe des "PMDI" wird bei 80 °C nachgerührt, um die Reaktion zu vervollständigen. Die vollständige Umsetzung (Freiheit von durch IR-Spektroskopie nachweisbaren Isocyanatgruppen) wird mittels FT-IR überprüft.

Beispiel 2: Allgemeine Arbeitsvorschrift II: Alternative Sythesemethode der cyclischen Carbonatharze am Beispiel des 4,4'-Diphenylmethandiurethandiglycerincarbonates mit Trimethylolpropantriglycidylether und Neopentylglykoldiglycidylether als Reaktivverdünner-Mischung:

In einem 120 mL Plastikbecher mit Schraubverschluss werden Trimethylolpropantriglycidylether, Neopentylglykoldiglycidylether und Glycerincarbonat in den in Tab. 3 (Beispiel 4) genannten Mengen vorgelegt. Nach ordentlicher Durchmischung wird das "PMDI" bei RT zugegeben und bis zur schlierenfreien Erscheinung erneut durchmischt. Anschließend wird der die Reaktionsmischung enthaltende Plastikbecher bei 40 °C gelagert, um die Reaktion zu vervollständigen. Die vollständige Umsetzung (Freiheit von durch IR-Spektroskopie nachweisbaren Isocyanatgruppen) wird mittels FT-IR überprüft.

Beispiel 3: Rezepturen für Carbonatharze I:

Die Rezeptur für die nach Beispiel 1 hergestellten Harze lautet:

Tabelle1: Rezeptur Carbonatharze mit Trimethylolpropantriglycidylether als Reaktivverdünner (hier: CVV-F-1 mit Isocyanat- bzw. Carbonat-Fkt.: 2,2)

| Bezeichnung | Einwaage m [g] | Gew.% |
|---|---|---|
| Trim | 57,81 | 60,00 |
| Glycerincarbonat | 18,54 | 19,24 |
| "PMDI" | 20,00 | 20,76 |

Beim "PMDI" handelt es sich um eine Abmischung aus MDI (Isocyanat-Funktionalität 2) und PMDI (Isocyanat-Funktionalität 3,2) und/oder monofunktionellem Isocyanat (Isocyanat-Funktionalität 1,0) entsprechend Tabelle 2.

MDI: Diphenylmethandiisocyanat-Isomerengemisch, Molekulargewicht 250 g/mol, Isocyanat-Funktionalität 2 (Herstellerangaben)
PMDI: Diphenylmethandiisocyanat mit Isomeren und höherfunktionellen Homologen, Molekulargewicht 430 g/mol, Isocyanat-Funktionalität 3,2 (Herstellerangaben)
p-TSI: p-Toluolsulfonylisocyanat, Molekulargewicht 197,21 g/mol, Isocyanat-Funktionalität 1 Trim: Trimethylolpropantriglycidylether (technisches Produkt), herstellbedingt können hier auch Diglycidylether und/oder Monoglycidylether des Trimethylolpropans und/oder höhere Homologe vorliegen

Tabelle 2: Abmischungen "PMDI" (evtl. mögliche Phenylmethylen-Einheiten bleiben unberücksichtigt)

| Bezeichnung | p-TSI [%] | MDI [%] | PMDI [%] | I- bzw. C-Fkt. |
|---|---|---|---|---|
| CVV-F-33 | 44,10 | 55,90 | - | 1,5 |
| CVV-F-1 bzw. CVV-F-22 | - | 74,40 | 25,60 | 2,2 |
| CVV-F-2 bzw. CVV-F-23 | - | 53,76 | 46,24 | 2,4 |
| CVV-F-3 bzw. CVV-F-24 | - | 36,76 | 63,24 | 2,6 |
| CVV-F-4 bzw. CVV-F-25 | - | 22,52 | 77,48 | 2,8 |

(fortgesetzt)

| Bezeichnung | p-TSI [%] | MDI [%] | PMDI [%] | I- bzw. C-Fkt. |
|---|---|---|---|---|
| CVV-F-5 bzw. CVV-F-26 | - | 10,42 | 89,58 | 3,0 |
| CVV-F-6 bzw. CVV-F-27 | - | 100,00 | 0,00 | 2,0 |
| CVV-F-7 bzw. CVV-F-28 | - | 0,00 | 100,00 | 3,2 |
| I-bzw. C-Fkt.: mittlere Isocyanat- bzw. Carbonat-Funktionalität | | | | |

Der Anteil des Reaktivverdünners von 60 Gew. % wird in den jeweiligen Rezepturen (CVV-F-1 bis CVV-F-7 bzw. CVV-F-33) beibehalten.

Beispiel 4: Rezepturen für Carbonatharze II mit Gesamtfunktionalität 2,7:

Die Rezeptur für die nach Beispiel 2 hergestellten Harze lautet:

Tabelle3: Rezeptur Carbonatharze mit Trimethylolpropantriglycidylether und Neopentylglykoldiglycidylether als Reaktivverdünner-Mischung (hier: CVV-F-22 mit Isocyanat- bzw. Carbonat-Fkt.: 2,2)

| Bezeichnung | Einwaage m [g] | Gew.% |
|---|---|---|
| Trim | 52,81 | 54,81 |
| Neopentylglykoldiglycidylether | 5,00 | 5,19 |
| Glycerincarbonat | 18,54 | 19,24 |
| "PMDI" | 20,00 | 20,76 |

Die Anteile an Trimethylolpropantriglycidylether und Neopentylglykoldiglycidylether der Reaktivverdünner-Mischung wird so gewählt, dass eine Gesamtfunktionalität des Systems (Carbonat-Funktionalitäten + Glycidyl-Funktionalitäten) von 2,7 gewährleistet bleibt.
Der Anteil der Reaktivverdünner-Mischung von 60 Gew. % wird in den jeweiligen Rezepturen (CVV-F-22 bis CVV-F-28) ebenfalls beibehalten.
Beim "PMDI" handelt es sich um eine Abmischung aus MDI (Isocyanat-Funktionalität 2) und PMDI (Isocyanat-Funktionalität 3,2) entsprechend Tabelle 2.

Beispiel 5: vereinfachte Mörtelrezeptur zur Durchführung für Setzversuche

Folgende Bestandteile werden in einer vereinfachten Mörtelrezeptur eingesetzt:

Tabelle 4: vereinfachte Mörtelrezeptur am Beispiel CVV-F-1

| Bezeichnung | Einwaage m [g] | Gew. % |
|---|---|---|
| CVV-F-1 | 35,20 | 55,00 |
| Zement | 28,80 | 45,00 |

Als Härter wird MXDA (meta-Xylylendiamin) in, zuvor mittels DSC-Messung - zur Erlangung möglichst hoher Tg Werte - ermittelten Mengen beispielsweise stöchiometrisch, oder insbesondere in überstöchiometrischen Mengen, eingesetzt.
Die Messung der Glasübergangstemperatur Tg (ein indirektes Maß u.a. für die Wärmeformbeständigkeit) erfolgt mittels Dynamischer Differenzkalorimetrie (DSC) in Anlehnung an ISO 11357-2 an Proben, die 24 h ausgehärtet werden.
Zur Berechnung des C-EP - Äquivalentgewichtes wird die Carbonat-Funktionalität mit der Epoxid-Funktionalität gleichgesetzt (Berechnung siehe Beispiel 6).

Tabelle 5-I: Verbundspannungen CW-F-33, CVV-F-1 bis CVV-F-7

| Bezeichnung | C-Fkt. | Verbundspannung [N/mm$^2$] |
|---|---|---|
| CVV-F-33 | 1,5 | 28 |
| CVV-F-1 | 2,2 | 31 |
| CVV-F-2 | 2,4 | 31 |
| CVV-F-3 | 2,6 | 30 |
| CVV-F-4 | 2,8 | 32 |
| CVV-F-5 | 3,0 | 32 |
| CVV-F-6 | 2,0 | 28 |
| CVV-F-7 | 3,2 | 32 |

Aus Tabelle 5-I wird ersichtlich, dass die Verbundspannungen mit einer Erhöhung der Funktionalität des Carbonatharzes tendenziell besser werden, bzw. die Verbundspannungen über den gesamten Funktionalitätsbereich konstant hoch liegen.

Setzversuche bzw. Auszugswerte: Die Verbundspannung wird ermittelt durch Auszugsversuche von Ankerstangen M12 aus Beton (C20/C25) mit einer Setztiefe von 72 mm und einem Bohrlochdurchmesser von 14 mm nach einer Aushärtezeit von 24 h bei RT.

Tabelle 5-II: materialspezifische Kenndaten CVV-F-22 bis CW-F-28

| Bezeichnung | C-Fkt. | GFkt | Verbundspannung [N/mm$^2$] | Viskosität [mPas] | w (CO2) [%] | Bemerkung |
|---|---|---|---|---|---|---|
| CVV-F-22 | 2,2 | 2,7 | 28 | 424300 | 7,18 | kristallin |
| CVV-F-23 | 2,4 | 2,7 | 29 | 257200 | 7,12 | kristallin |
| CVV-F-24 | 2,6 | 2,7 | 28 | 177500 | 7,08 | klar |
| CVV-F-25 | 2,8 | 2,7 | 28 | 130300 | 7,04 | klar |
| CVV-F-26 | 3,0 | 2,7 | 28 | 103900 | 7,00 | klar |
| CVV-F-27 | 2,0 | 2,7 | 28 | n.m. | 7,24 | kristallin |
| CVV-F-28 | 3,2 | 2,7 | 27 | 98630 | 6,97 | klar |
| n.m.: nicht messbar | | | | | | |

Die Tabelle 5-II zeigt wiederum, dass die Verbundspannungen im gesamten Carbonat-Funktionalitätsbereich konstant hoch liegen. Außerdem wird ersichtlich, dass mit der Erhöhung der Isocyanat- bzw. Carbonat-Funktionalität die Viskosität erniedrigt wird und die Kristallisationsneigung, die vermutlich auf die Ausbildung von Wasserstoffbrücken zurückzuführen ist, unterdrückt wird. Kristalline oder teilkristalline Harze können aufgrund ihrer schweren Auspressbarkeit zur Verwendung in Kartuschen-Injektionssystemen ungeeignet sein. Die erfindungsgemäß verwendbaren Cyclocarbonatharze können dementsprechend als Harze für Kartuschen-Injektionssysteme verwendet werden, ohne an Auszugskraft einzubüßen. Zusätzlich zu diesen beiden Entdeckungen kann der Anteil an chemisch fixiertem $CO_2$, was der Verminderung von Treibhausgasen Beitrag leistet, hoch gehalten werden (siehe Tabelle 5-II). Der Anteil an chemisch fixiertem $CO_2$ sinkt leicht mit der Erhöhung der Carbonat-Funktionalität, da das für die Funktionalitätserhöhung verwendete PMDI einen geringeren NCO-Gehalt aufweist.

Die Tabelle 5-II belegt außerdem die Aussage, dass die erwähnten vorteilhaften Eigenschaften auch mit sehr hohen Verbundspannungen zu vereinbaren sind.

Beispiel 6: Berechnung des C-EP - Äquivalentgewichtes am Beispiel CVV-F-1

Die nachfolgende Tabelle 6 zeigt exemplarisch die CVV-F-1 - Rezeptur:

| Bezeichnung | Einwaage m [g] | Gew.% |
|---|---|---|
| Trim | 57,81 | 60,00 |

(fortgesetzt)

| Bezeichnung | Einwaage m [g] | Gew.% |
|---|---|---|
| Glycerincarbonat | 18,54 | 19,24 |
| "PMDI" | 20,00 | 20,76 |
| Gesamt | 96,35 | 100,00 |

Zur Berechnung des **C**arbonat-**E**poxid - Äquivalentgewichtes (C-EP - Äq) werden die Mole an Carbonat-Funktionalitäten und die Mole an Glycidyl-/Epoxy-Funktionalitäten des Trimethylolpropantriglycidylethers addiert und das Gesamtgewicht durch die addierten Mole der Funktionalitäten dividiert:

$$n\ (\text{Carbonat}) = m\ (\text{Glycerincarbonat}) / M\ (\text{Glycerincarbonat}) = 18{,}54\ g / 118{,}0\ g/mol = 0{,}157\ mol$$

$$n\ (\text{Glycidyl}) = m\ (\text{Trim}) / \text{EP-Äquivalentgewicht} = 57{,}81\ g / 145\ g/mol = 0{,}399\ mol$$

$$\text{C-EP} - \text{Äq} = m\ (\text{Gesamt}) / [n\ (\text{Carbonat}) + n\ (\text{Glycidyl})] = 96{,}35\ g / 0{,}556\ mol = 173\ g/mol$$

m bedeutet jeweils das Gewicht (in g), n die Stoffmenge (in Mol).

Beispiel 7: Berechnung des chemisch fixierten $CO_2$-Gehaltes am Beispiel CVV-F-1:

Anteil $CO_2$ in Glycerincarbonat:

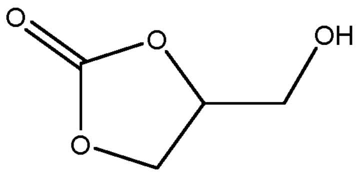

w $(CO_2)$Glycerincarbonat = M $(CO_2)$ / M (Glycerincarbonat) = 44,01 g/mol / 118,0 g/mol = 0,373 Der Gewichtsanteil an chemisch fixiertem $CO_2$ in Glycerincarbonat beträgt 37,30 %. Der Gewichtsanteil an chemisch fixiertem $CO_2$ in 100 g CVV-F-1 beträgt:

$$\text{Gew. \% } (CO_2)_{\text{CVV-F-1}} = (100\ g \times \text{Gew. \% (Glycerincarbonat)} / 100) \times w\ (CO_2)_{\text{Glycerincarbonat}} = 7{,}18\ \%.$$

In 100 g CVV-F-1 sind somit 7,18 g (3,66 L) $CO_2$ fixiert.

Beispiel 8: Einfluss einer Acetoacetato-Verbindung auf die Verbundspannung

Anhand einer vereinfachten Mörtelrezeptur gemäß Tabelle 4 wird der Einfluss einer Acetoacetato-Verbindung auf die Verbundspannung ermittelt. Die Setzversuche werden entsprechend der in Beispiel 5 beschriebenen Methode durchgeführt. Die Rezepturen der eingesetzten Carbonatharze sind entsprechend der Tabelle 6 formuliert, wobei als "PMDI" Desmodur VKS 20 eingesetzt wird (CVV-01-04). Bei CVV-23-01 werden 5 Gew.% TRIM durch Lonzamon AATMP ersetzt. Die nachfolgende Tabelle 7 zeigt die ermittelten Verbundspannungen.

Tabelle 7: Verbundspannungen Carbonatharze (ohne und mit Acetoacetato-Verbindung)

| Bezeichnung | C-Fkt. | Verbundspannung [N/mm2] |
|---|---|---|
| CVV-01-04 (ohne) | 2,9 | 28 |

(fortgesetzt)

| Bezeichnung | C-Fkt. | Verbundspannung [N/mm2] |
|---|---|---|
| CVV-23-01 (mit) | 2,9 | 31 |

| | |
|---|---|
| Desmodur VKS 20: | Gemisch von Diphenylmethan-4,4'-diisocyanat mit Isomeren und höheren Homologen, Isocyanat-Funktionalität: 2,9 (Herstellerangabe), Bayer Material Science |
| Lonzamon AATMP: | Trimethylolpropantriacetoacetat (Acetylacetonat-Trimer), Lonza |

Die Tabelle 7 zeigt, dass durch den Einsatz einer Acetoacetato-Verbindung die Verbundspannung gesteigert werden kann (hier beispielhaft dargestellt durch Zugabe von 5 Gew.% Lonzamon AATMP). Durch Erhöhen des Anteils an AATMP und entsprechendem Ersetzen von TRIM gemäß Tabelle 8 der Reaktivverdünner wird ein Maximum an Verbundspannung durchlaufen.

Tabelle 8: Abmischungen TRIM mit AATMP als Reaktivverdünner

| | TRIM [Gew.%] | AATMP [Gew.%] |
|---|---|---|
| CVV-01-04 | 60,00 | 0,00 |
| CVV-23-01 | 55,00 | 5,00 |
| CVV-23-02 | 50,00 | 10,00 |
| CVV-23-03 | 30,00 | 30,00 |
| CVV-23-04 | 10,00 | 50,00 |
| CVV-23-05 | 0,00 | 60,00 |

Beispiel 9: Vergleichsbeispiel zur Abgrenzung gegenüber WO 2006/010408

Wie eingangs erläutert, beschreibt die WO 2006/010408 isocyanatgruppenfreie Umsetzungsprodukte linearer Polyurethanpräpolymere auf Basis von Diphenylmethandiisocyanat (MDI) mit Glycerincarbonat. Derartig hergestellte Carbonatharze können zwar als Kleb- und Dichtstoff Einsatz finden, jedoch sind sie aufgrund von zu hohen Viskositäten nicht für die Verwendung als kalthärtendes 2 K-Kartuschen-Injektionssystem, im Bereich der Befestigungstechnik, zumutbar. Die nachfolgende Tabelle 9 zeigt die ermittelten Viskositäten im Vergleich zu den erfindungsgemäß verwendbaren Carbonatharzen.

Tabelle 9: Viskositätsvergleich erfindungsgemäß verwendbarer Carbonatharze mit denen aus WO 2006/010408

| Bezeichnung | Viskosität (gerundet) [Pas] |
|---|---|
| Vergleichsbeispiel-V-1 | 1540 bei 60 °C |
| Vergleichsbeispiel-V-2 | 3286 bei 60 °C |
| CVV-F-28 | 99 bei 23 °C |

Die Viskositäten werden gemessen mit einem Rheometer AR2000 der Firma TA Instruments mit einer Platte-Platte-Geometrie, Durchmesser 25 mm, Spalt 1000 μm im Flowversuch mit einem Drehmoment von 50.000 μN·m bei 23 bzw. 60 °C.

Die Vergleichsbeispiele werden gemäß dem in WO 2006/010408 beschriebenen Beispiel 1 hergestellt. Für die Präpolymerbildung werden entsprechende Polyole verwendet.

Vergleichsbeispiel-V-1: verwendete Polyole

| | |
|---|---|
| Polyol A1: | Baygal VP.PU70RE30; flüssiges Polyetherpolyol der Firma Bayer Material Science mit einer OH-Zahl von 56 |
| Polyol B1: | Voranol CP 1055; trifunktionelles Polypropylenglykol der Firma Dow mit einer OH-Zahl von 156 |
| Polyol C: | Baycoll AD 3040; flüssiges Polyesterpolyol der Firma Bayer Material Science mit einer OH-Zahl von 43 |

Vergleichsbeispiel-V-2: verwendete Polyole

| | |
|---|---|
| Polyol A2: | Baycoll AD 2070; flüssiges Polyesterpolyol der Firma Bayer Material Science mit einer OH-Zahl von 55 |
| Polyol B2: | Voranol 1010L; difunktionelles Polypropylenglykol der Firma Dow mit einer OH-Zahl von 110 |
| Polyol C: | Baycoll AD 3040; flüssiges Polyesterpolyol der Firma Bayer Material Science mit einer OH-Zahl von 43 |

Der ermittelte Viskositätsunterschied (bei 23 °C waren die Vergleichsbeispiele V1 und V2 nicht messbar) zusammen mit den in den Beispielen der WO angegebenen Schmelzviskositäten bei 125 °C, sowie die Maßgabe, dass es sich in der WO um Carbonatharze mit einem mittleren Molekulargewicht ($M_n$) von ≥ 1000 g/mol handelt, belegen die Andersartigkeit der beiden Anwendungen bzw. Erfindungen.

## Patentansprüche

1. Verwendung eines Kunstharz-Befestigungssystems auf Basis cyclischer Carbonatharze zur Befestigung von Verankerungsmitteln in Löchern oder Spalten, **dadurch gekennzeichnet, dass** das Kunstharzbefestigungssysstem als cyclisches Carbonatharz mindestens eines mit einer durchschnittlichen Funktionalität von 1,5 oder mehr als 1,5 cyclischen Carbonatgruppen je Molekül beinhaltet.

2. Verwendung nach Anspruch 1, **dadurch gekennzeichnet, dass** im Kunstharzbefestigungssystem das Carbonatharz eine durchschnittliche Funktionalität von 2,1 bis 5, zum Beispiel von 2,2 bis 4, vorteilhaft z.B. von 2,3 bis 3,5, cyclischen Carbonatgruppen je Molekül aufweist, wobei vorzugsweise
das Molekulargewicht der cyclischen Carbonatharze unter 1000 g/mol, insbesondere bei ≤ 990, z.B. ≤ 950, wie bei 800 g/mol oder niedriger liegt.

3. Verwendung nach einem der Ansprüche 1 bis 2, **dadurch gekennzeichnet, dass** das Kunstharz-Befestigungssystem als cyclisches Carbonatharz ein solches beinhaltet, welches durch Umsetzung hydroxyfunktioneller cyclischer Carbonate mit Isocyanaten mit einer durchschnittlichen Funktionalität von 1,5 oder größer als 1,5, beispielsweise von 2,1 bis 5, zum Beispiel von 2,2 bis 4, vorteilhaft z.B. von 2,3 bis 3,5, Isocyanatgruppen je Molekül erhältlich ist.

4. Verwendung nach einem der Ansprüche 1 bis 3 des Kunstharz-Befestigungssystems in Form eines Mehrkomponentensystems, insbesondere eines Zwei-Komponentensystems oder -kits mit einer Komponente (A), welche das oder die cyclischen Carbonatharze beinhaltet, und einer Härterkomponente (B).

5. Verwendung nach einem der Ansprüche 1 bis 4 oder 8, **dadurch gekennzeichnet, dass** es im Härter ein Di- oder Polyamin und/oder ein Di- oder Polythiol beinhaltet.

6. Kunstharz-Befestigungssystem auf Basis cyclischer Carbonatharze wie in einem der Ansprüche 1 bis 4 definiert, **dadurch gekennzeichnet, dass** es einen funktionelle Gruppen aufweisenden Reaktiwerdünner in Form einer Mischung aus einem difunktionellen und einem trifunktionellen vernetzenden Reaktivverdünner beinhaltet.

7. Kunstharz-Befestigungssystem nach Anspruch 6, **dadurch gekennzeichnet, dass** es als Reaktivverdünner mindestens einen Triglycidylether beinhaltet.

8. Kunstharz-Befestigungssystem nach Anspruch 6 oder 7 in Form eines Zwei-Komponentensystems, **dadurch gekennzeichnet, dass** es als Reaktivverdünner eine Mischung aus mindestens einem Diglycidylether und mindestens

einem Triglycidylether beinhaltet und worin die mittlere Epoxy-Funktionalität der Reaktivverdünnermischung größer als 2 ist.

9. Kunstharz-Befestigungssystem wie in Anspruch 4 definiert oder Kunstharz-Befestigungssystem nach Anspruch 6, **dadurch gekennzeichnet, dass** es einen oder mehrere cyclische Carbonatgruppen beinhaltende Reaktivverdünner beinhaltet.

10. Kunstharz-Befestigungssystem nach Anspruch 6, **dadurch gekennzeichnet, dass** es als Reaktivverdünner mindestens ein Triglycidylethercarbonat beinhaltet.

11. Kunstharz-Befestigungssystem nach Anspruch 6 oder 10, **dadurch gekennzeichnet, dass** es als Reaktivverdünner eine Mischung aus mindestens einem Diglycidylethercarbonat und/oder mindestens einem Triglycidylethercarbonat beinhaltet und worin die mittlere Carbonat-Funktionalität der Reaktivverdünnermischung größer als 2 ist.

12. Kunstharz-Befestigungssystem nach Anspruch 11, **dadurch gekennzeichnet, dass** es als Reaktivverdünner Glycerintriglycidylether(carbonat), Pentaerythrittetraglycidylether(carbonat) und/oder Trimethylolpropantriglycidylether(carbonat) oder Mischungen davon mit 1,4-Butandioldiglycidylether(carbonat), Cyclohexandimethanoldiglycidylether(carbonat), Neopentylglykoldiglycidylether(carbonat), Hexandioldiglycidylether(carbonat) und/oder Propylen-glykoldiglycidylether(carbonat) beinhaltet.

13. Kunstharz-Befestigungssystem nach Anspruch 6, **gekennzeichnet durch** einen Gehalt an Mono-, Bis-, Tris- und/oder Tetraacetoacetatverbindung, insbesondere Trimethylolpropantrisacetoacetat und/oder Trimethylolethantrisacetoacetat, wobei es vorzugsweise neben der Acetoacetatverbindung ein oder mehrere Metallverbindungen enthält, insbesondere als Metallverbindung Erdalkalioxide, vorzugsweise Calciumoxid und/oder Magnesiumoxid.

14. Kunstharz-Befestigungssystem nach Anspruch 6, **dadurch gekennzeichnet, dass** es als cyclisches Carbonatharz ein Reaktionsprodukt von Glycerincarbonat und einem Isocyanat, wobei als Isocyanat eine Mischung aus Diphenylmethandiisocyanat-Isomerengemisch mit einer durchschnittlichen Isocyanat-Funktionalität je Molekül von 2 und/oder Diphenylmethandiisocyanat mit Isomeren und höherfunktionellen Homologen und einer durchschnittlichen Isocyanat-Funktionalität je Molekül von 3,2 und/oder monofunktionalenIsocyanaten mit einer durchschnittlichen Isocyanat-Funktionalität je Molekül von 1, beinhaltet und als Reaktivverdünner einen Triglycidylether und/oder einen Diglycidylether, insbesondere Trimethylolpropantriglycidylether und/oder Neopentylglykoldiglycidylether beinhaltet.

15. Kunstharz-Befestigungssystem nach einem der Ansprüche 6 bis 8 oder 10 bis 14, **dadurch gekennzeichnet, dass** es im Härter ein Di- oder Polyamin und/oder ein Di- oder Polythiol beinhaltet.

## Claims

1. Use of a synthetic resin fixing system based on cyclic carbonate resins for fixing anchoring means in holes or crevices, **characterised in that** the synthetic resin fixing system comprises as cyclic carbonate resin at least one having an average functionality of 1.5 or more than 1.5 cyclic carbonate groups per molecule.

2. Use according to claim 1, **characterised in that** in the synthetic resin fixing system the carbonate resin has an average functionality of from 2.1 to 5, for example from 2.2 to 4, advantageously, for example, from 2.3 to 3.5, cyclic carbonate groups per molecule, the molecular weight of the cyclic carbonate resins preferably being below 1000 g/mol, especially being $\leq$ 990, for example $\leq$ 950, such as 800 g/mol or less.

3. Use according to either one of claims 1 and 2, **characterised in that** the synthetic resin fixing system comprises as cyclic carbonate resin one which is obtainable by reaction of hydroxy-functional cyclic carbonates with isocyanates having an average functionality of 1.5 or greater than 1.5, for example from 2.1 to 5, for example from 2.2 to 4, advantageously, for example, from 2.3 to 3.5, isocyanate groups per molecule.

4. Use according to any one of claims 1 to 3 of the synthetic resin fixing system in the form of a multi-component system, especially a two-component system or kit having a component (A), which comprises the cyclic carbonate resin(s), and a hardener component (B).

5. Use according to any one of claims 1 to 4 or 8, **characterised in that** it comprises in the hardener a di- or poly-amine and/or a di- or poly-thiol.

6. Synthetic resin fixing system based on cyclic carbonate resins as defined in any one of claims 1 to 4, **characterised in that** it comprises a reactive diluent having functional groups in the form of a mixture of a difunctional and a trifunctional crosslinking reactive diluent.

7. Synthetic resin fixing system according to claim 6, **characterised in that** it comprises as reactive diluent at least one triglycidyl ether.

8. Synthetic resin fixing system according to claim 6 or 7 in the form of a two-component system, **characterised in that** it comprises as reactive diluent a mixture of at least one diglycidyl ether and at least one triglycidyl ether and wherein the average epoxy functionality of the reactive diluent mixture is greater than 2.

9. Synthetic resin fixing system as defined in claim 4 or a synthetic resin fixing system according to claim 6, **characterised in that** it comprises one or more reactive diluents comprising cyclic carbonate groups.

10. Synthetic resin fixing system according to claim 6, **characterised in that** it comprises as reactive diluent at least one triglycidyl ether carbonate.

11. Synthetic resin fixing system according to claim 6 or 10, **characterised in that** it comprises as reactive diluent a mixture of at least one diglycidyl ether carbonate and/or at least one triglycidyl ether carbonate and wherein the average carbonate functionality of the reactive diluent mixture is greater than 2.

12. Synthetic resin fixing system according to claim 11, **characterised in that** it comprises as reactive diluent glycerol triglycidyl ether (carbonate), pentaerythritol tetraglycidyl ether (carbonate) and/or trimethylolpropane triglycidyl ether (carbonate) or mixtures thereof with 1,4-butanediol diglycidyl ether (carbonate), cyclohexanedimethanol diglycidyl ether (carbonate), neopentyl glycol diglycidyl ether (carbonate), hexanediol diglycidyl ether (carbonate) and/or propylene glycol diglycidyl ether (carbonate).

13. Synthetic resin fixing system according to claim 6, **characterised by** a content of mono-, bis-, tris- and/or tetra-acetoacetate compound, especially trimethylolpropane trisacetoacetate and/or trimethylolethane trisacetoacetate, wherein in addition to the acetoacetate compound it preferably contains one or more metal compounds, especially containing as metal compound alkaline earth oxides, preferably calcium oxide and/or magnesium oxide.

14. Synthetic resin fixing system according to claim 6, **characterised in that** it comprises as cyclic carbonate resin a reaction product of glycerol carbonate and an isocyanate, comprising as isocyanate a mixture of diphenylmethane diisocyanate isomeric mixture having an average isocyanate functionality per molecule of 2 and/or diphenylmethane diisocyanate with isomers and higher-functional homologues and an average isocyanate functionality per molecule of 3.2 and/or monofunctional isocyanates having an average isocyanate functionality per molecule of 1, and comprising as reactive diluent a triglycidyl ether and/or a diglycidyl ether, especially trimethylolpropane triglycidyl ether and/or neopentyl glycol diglycidyl ether.

15. Synthetic resin fixing system according to any one of claims 6 to 8 or 10 to 14, **characterised in that** it comprises in the hardener a di- or poly-amine and/or a di- or poly-thiol.

## Revendications

1. Utilisation d'un système de fixation en résine synthétique à base de résines de carbonate cyclique pour fixer des moyens d'ancrage dans des trous ou des fentes, **caractérisée en ce que** le système de fixation en résine synthétique contient comme résine de carbonate cyclique au moins une résine ayant une fonctionnalité moyenne de 1,5 ou plus de 1,5 groupes carbonate cycliques par molécule.

2. Utilisation selon la revendication 1, **caractérisée en ce que** dans le système de fixation en résine synthétique, la résine de carbonate présente une fonctionnalité moyenne de 2,1 à 5, par exemple de 2,2 à 4, avantageusement par exemple de 2,3 à 3,5, groupes carbonate cycliques par molécule, de préférence le poids moléculaire des résines de carbonate cyclique étant inférieur à 1000 g/mole, en particulier $\leq$ 990, par exemple $\leq$ 950, comme égal à 800 g/mole ou moins.

3. Utilisation selon l'une des revendications 1 à 2, **caractérisée en ce que** le système de fixation en résine synthétique contient comme résine de carbonate cyclique une résine qui peut être obtenue en faisant réagir des carbonates cycliques hydroxyfonctionnels avec des isocyanates ayant une fonctionnalité moyenne de 1,5 ou supérieure à 1,5, par exemple de 2,1 à 5, comme de 2,2 à 4, avantageusement par exemple de 2,3 à 3,5, groupes isocyanate par molécule.

4. Utilisation selon l'une des revendications 1 à 3 du système de fixation en résine synthétique sous la forme d'un système à plusieurs composants, en particulier d'un système ou d'un kit à deux composants avec un composant (A) qui contient la ou les résines de carbonate cyclique et un composant durcisseur (B).

5. Utilisation selon l'une des revendications 1 à 4 ou 8, **caractérisée en ce qu'**une di- ou polyamine et/ou un di- ou polythiol sont contenus dans le durcisseur.

6. Système de fixation en résine synthétique à base de résines de carbonate cyclique telles que définies dans l'une des revendications 1 à 4, **caractérisé en ce qu'**il contient un diluant réactif présentant des groupes fonctionnels sous la forme d'un mélange d'un diluant réactif réticulant difonctionnel et d'un diluant réactif réticulant trifonctionnel.

7. Système de fixation en résine synthétique selon la revendication 6, **caractérisé en ce qu'**il contient au moins un éther triglycidylique comme diluant réactif.

8. Système de fixation en résine synthétique selon la revendication 6 ou 7 sous la forme d'un système à deux composants, **caractérisé en ce qu'**il contient comme diluant réactif un mélange d'au moins un éther diglycidylique et d'au moins un éther triglycidylique et dans lequel la fonctionnalité époxy moyenne du mélange de diluants réactifs est supérieure à 2.

9. Système de fixation en résine synthétique tel que défini dans la revendication 4 ou système de fixation en résine synthétique selon la revendication 6, **caractérisé en ce qu'**il contient un ou plusieurs diluants réactifs contenant des groupes carbonate cycliques.

10. Système de fixation en résine synthétique selon la revendication 6, **caractérisé en ce qu'**il contient au moins un carbonate d'éther triglycidylique comme diluant réactif.

11. Système de fixation en résine synthétique selon la revendication 6 ou 10, **caractérisé en ce qu'**il contient comme diluant réactif un mélange d'au moins un carbonate d'éther diglycidylique et/ou au moins un carbonate d'éther triglycidylique et dans lequel la fonctionnalité carbonate moyenne du mélange de diluants réactifs est supérieure à 2.

12. Système de fixation en résine synthétique selon la revendication 11, **caractérisé en ce qu'**il contient comme diluant réactif de l'éther(carbonate) triglycidylique de glycérol, de l'éther(carbonate) tétraglycidylique de pentaérythritol et/ou de l'éther(carbonate) triglycidylique de triméthylolpropane ou leurs mélanges avec du 1,4-éther(carbonate) diglycidylique de butanediol, de l'éther(carbonate) diglycidylique de cyclohexanediméthanol, de l'éther(carbonate) diglycidylique de néo-pentylglycol, de l'éther(carbonate) diglycidylique d'hexanediol et/ou de l'éther(carbonate) diglycidylique de propylèneglycol.

13. Système de fixation en résine synthétique selon la revendication 6, **caractérisé par** une teneur en composé mono-, bis-, tris- et/ou tétraacétoacétate, en particulier en trisacétoacétate de triméthylolpropane et/ou trisacétoacétate de triméthyloléthane, et contenant de préférence, en plus du composé acétoacétate, un ou plusieurs composés métalliques, en particulier des oxydes alcalino-terreux, de préférence de l'oxyde de calcium et/ou de l'oxyde de magnésium comme composé métallique.

14. Système de fixation en résine synthétique selon la revendication 6, **caractérisé en ce qu'**il contient comme résine de carbonate cyclique un produit de réaction du carbonate de glycérol et d'un isocyanate, comme isocyanate un mélange de mélange d'isomères de diphénylméthane diisocyanate ayant une fonctionnalité isocyanate moyenne par molécule de 2 et/ou de diphénylméthane diisocyanate avec des isomères et des homologues fonctionnels supérieurs et une fonctionnalité isocyanate moyenne par molécule de 3,2 et/ou d'isocyanates monofonctionnels ayant une fonctionnalité isocyanate moyenne par molécule de 1, et contient comme diluant réactif un éther triglycidylique et/ou un éther diglycidylique, en particulier de l'éther triglycidylique de triméthylolpropane et/ou de l'éther diglycidylique de néopentylglycol.

**15.** Système de fixation en résine synthétique selon l'une des revendications 6 à 8 ou 10 à 14, **caractérisé en ce qu'**il contient une di- ou polyamine et/ou un di- ou polythiol dans le durcisseur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3529263
- DE 3600602
- WO 8403701 A
- US 3072613 A
- EP 0703230 A
- WO 2006010408 A
- US 20100137507 A
- WO 2014033045 A
- DE OS2611087 A
- DE 10104437
- WO 2005090433 A
- EP 0645408 A
- DE 102013113465
- EP 0824124 A
- DE 69827788 T2


### In der Beschreibung aufgeführte Nicht-Patentliteratur


- **BECKER, G.W. ; BRAUN, D. ; OERTEL, G.** Polyurethane Kunststoff-Handbuch. Carl Hanser Verlag, 1993, vol. 7
- **LEE H ; NEVILLE K.** Handbook of Epoxy Resins. McGraw-Hill, 1982